(19) Europäisches Patentamt
European Patent Office
Office européen des brevets

(11) Veröffentlichungsnummer: **0 282 069 B1**

(12) **EUROPÄISCHE PATENTSCHRIFT**

(45) Veröffentlichungstag der Patentschrift: **09.06.93**

(51) Int. Cl.[5]: **C08G 18/20**, C08G 59/68, C07D 471/08, C07D 209/52

(21) Anmeldenummer: **88103856.6**

(22) Anmeldetag: **11.03.88**

Die Akte enthält technische Angaben, die nach dem Eingang der Anmeldung eingereicht wurden und die nicht in dieser Patentschrift enthalten sind.

# (54) Bicyclische Aminkatalysatoren.

(30) Priorität: **12.03.87 DE 3707911**

(43) Veröffentlichungstag der Anmeldung:
**14.09.88 Patentblatt 88/37**

(45) Bekanntmachung des Hinweises auf die Patenterteilung:
**09.06.93 Patentblatt 93/23**

(84) Benannte Vertragsstaaten:
**AT BE CH DE ES FR GB IT LI NL SE**

(56) Entgegenhaltungen:
**US-A- 3 036 021**
**US-A- 3 539 660**

**THE JOURNAL OF THE AMERICAN CHEMICAL SOCIETY, Band 107, 20. März 1985, Seiten 1768-1769, American Chemical Society, Washington, US; S.D. LARSEN et al.: "Aza Diels-Alder reactions in aqueous solution: cyclocondensation of dienes with simple iminium salts generated under Mannich conditions"**

(73) Patentinhaber: **RWE-DEA AKTIENGESELLSCHAFT FÜR MINERALOEL UND CHEMIE**
**Überseering 40**
**W-2000 Hamburg 60(DE)**

(72) Erfinder: **Gluzek, Karl-Heinz, Dr.**
**Die Schraag 11**
**W-4234 Alpen(DE)**
Erfinder: **Humbert, Heiko, Dr.**
**Hugo-Klemm-Strasse 23**
**W-2100 Hamburg 90(DE)**

(74) Vertreter: **Schupfner, Gerhard D.**
**Müller, Schupfner & Gauger Karlstrasse 5**
**Postfach 14 27**
**W-2110 Buchholz/Nordheide (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 282 069 B1

THE JOURNAL OF ORGANIC CHEMISTRY, Band 52, 25. Dezember 1987, Seiten 5746-5749, American Chemical Society, Washington, US; P.A. GRIECO et al.: "Immonium ion based synthetic methodology: A novel method for the N-methylation of dipeptides and amino acid derivatives via retro aza Diels-Alder reactions"

THE JOURNAL OF THE AMERICAN CHEMICAL SOCIETY, Band 100, 1. März 1978, Seiten 1503-1505, American Chemical Society, Washington, US; F.M. MENGER et al.: "Exo-endo vs. equatorial-axial equilibria. Assessment of steric crowding in the endo cavity"

TETRAHEDRON, Band 21, Nr. 10, Oktober 1965, Seiten 2725-2734, Pergamon Press, Ltd, Oxford, GB; P.G. GASSMAN et al.: "The synthesis of 2-azabicyclo[2.2.1]heptanes by the Hofmann-Löffler-Freytag reaction"

## Beschreibung

Die vorliegende Erfindung bezieht sich auf neu 2-Azabicyclo[2.2.1]heptan- bzw. -heptenderivate sowie auf die Herstellung von substituierten 2-Azabicyclo-[2.2.1]hept-5-enen durch Umsetzung von Cyclopentadien mit Ammoniumsalzen bzw. den Salzen primärer Amine und Aldehyde zu den Salzen der 2-Azabicyclo-[2.2.1]hept-5-en-Derivate, Neutralisation der Salze und ggf. Hydrierung der Basen zu Derivaten des 2-Azabicyclo[2.2.1]-heptans und auf die Anwendung der Verfahrensprodukte als Aminkatalysatoren.

Es ist bekannt, N-Alkyl-2-azabicyclo[2.2.1]heptane durch Hofmann-Löffler-Freytag-Reaktion aus N-Alkylaminomethylcyclopentan-Verbindungen herzustellen, wobei die Synthese der Ausgangsverbindungen in mehreren Stufen aus Cyclopentancarbonsäure und einem primären Amin erfolgt, siehe P.G. Gassmann und D.C. Heckert, Tetrahedron 21, 2725 (1965). So kann z. B. das N-Ethyl-2-azabicyclo[2.2.1]heptan in einer Gesamtausbeute von 24 % hergestellt werden. Der beschriebene Herstellungsweg hat wegen der geringen Gesamtausbeute und des hohen Preises der als Ausgangsstoff dienenden Cyclopentancarbonsäure keinen Eingang in die Technik gefunden.

Es ist weiterhin bekannt, am Stickstoff substituierte Derivate des 2-Azabicyclo[2.2.1]hept-5-en durch Umsetzung von Iminiumsalzen mit Cyclopentadien herzustellen, siehe S.D. Larsen und P.A. Grieco, J. Am. Chem. Soc. 107, 1768 (1985). S.D. Larsen und P.A. Grieco führten die Reaktion bei Raumtemperatur oder leicht erhöhter Temperatur im Zweiphasensystem Wasser/Cyclopentadien durch, wobei Aminhydrochlorid und Formal in im Überschuß von 30 - 40 Mol % verwendet wurden.

Lineare, mono- oder bicyclische Amine sind als Katalysatoren für die Herstellung von Polyurethanen in die Technik eingeführt. Der Erfindung lag die Aufgabe zugrunde, neue bicyclische Amine herzustellen, die als Katalysatoren geeignet sind und die aus technisch leicht zugänglichen und kostengünstigen Rohstoffen synthetisiert werden können.

In Lösung der gestellten Aufgabe werden die Verbindungen gemäß Anspruch 1 als Katalysatoren sowie Verfahren gemäß Anspruch 2 zu deren Herstellung bereitgestellt.

Die Herstellung der 2-Azabicyclo[2.2.1]hept-5-ene und 2-Azabicyclo[2.2.1]heptane erfolgt nach an sich bekannten Syntheseverfahren der organischen Chemie. Als Rohstoffe dienen primäre Amine oder deren Salze mit starken Säuren, Carbonylverbindungen und Cyclopentadien. Als Carbonylkomponente wird Formaldehyd bevorzugt.

Das neue Verfahren wird in zwei Synthesestufen durchgeführt:
Im ersten Syntheseschritt wird das 2-Azabicyclo[2.2.1]hept-5-en-Derivat hergestellt. Hierzu wird ein primäres Amin mit einer starken Mineralsäure, wie Salzsäure, Schwefelsäure, Phosphorsäure oder einer starken organischen Säure wie p-Toluolsulfonsäure oder deren Mischungen neutralisiert, mit einer Carbonylverbindung, insbesondere Formalin, zum Iminiumsalz umgesetzt und nach Zugabe des Cyclopentadiens im Zweiphasensystem insbesondere bei 40 bis 45 °C zum 2-Azabicyclo-[2.2.1.]hept-5-en-Derivat umgesetzt.

Die Reaktion läuft nur im sauren pH-Bereich ab, und zwar bevorzugt in einen pH-Bereich zwischen 2 und 6, insbesondere in einem pH-Bereich zwischen 2 und 3.

Ammoniumsalze wie Ammoniumchlorid ergeben nur mäßige Ausbeuten, da Formalin alkylierend wirkt, sodaß ein destillativ schwer zu trennendes Gemisch von 2-Azabicyclo[2.2.1]hept-5-ene und N-Methyl-2-azabicyclo[2.2.1.]-hept-5-ene entsteht.

Aus dem Salz des 2-Azabicyclo[2.2.1]hept-5-en-Derivates wird das Amin durch Zugabe starker Basen wie Alkalihydroxide, Erdalkalioxide, Erdalkalihydroxide oder organischer Basen in Freiheit gesetzt.

In der zweiten Verfahrensstufe wird das 2-Azabicyclo[2.2.1]hept-5-en hydriert. Dabei ist es nicht erforderlich, das reine 2-Azabicyclo[2.2.1]hept-5-en-Derivat einzusetzen; stattdessen kann das Rohprodukt, das gegebenenfalls noch Wasser enthalten darf, direkt in an sich bekannter Weise in Gegenwart eines Katalysators hydriert werden.

Die Hydrierung erfolgt in der Regel unter Druck in Gegenwart eines Edelmetallkatalysators, insbesondere der Platingruppe und bevorzugt in Gegenwart von Palladium. Gegebenenfalls wird bei erhöhter Temperatur gearbeitet.

Zur Hydrierung können entweder die freien Basen oder unmittelbar die in Lösung anfallenden Salze eingesetzt werden. Bevorzugt werden die freien Basen hydriert. Das Verfahren kann ansatzweise oder kontinuierlich durchgeführt werden.

Sowohl das 2-Azabicyclo[2.2.1]hept-5-en als als auch das 2-Azabicyclo[2.2.1]heptan sind Feststoffe, siehe A. Heesing und W. Herdering, Chem. Ber. 116, 1081-1096 (1983). Alle anderen hergestellten Verbindungen sind Flüssigkeiten, die im Vakuum destilliert werden können.

Die 2-Azabicyclo[2.2.1]heptan-Derivat sind thermisch stabiler als die zugrundeliegenden 2-Azabicyclo-[2.2.1]hept-5-en-Derivat, die zum Teil in Umkehrung der Bildung wieder in die Ausgangsstoffe zerfallen. Die leichtflüchtigen Verbindungen sind durch einen aminartigen Geruch gekennzeichnet; hochsiedende 2-Azabicyclo[2.2.1]heptan-Derivat sind praktisch geruchlos.

Die nach dem beschriebenen Verfahren hergestellten Derivate des 2-Azabicyclo[2.2.1]heptans können weiter abgewandelt werden, sofern sie in der Seitenkette $R_1$ OH-Gruppen tragen. So kann z. B. die OH-Gruppe des N-(2-Hydroxyethyl-) bzw. des N-(2-Hydroxyethoxyethyl)-2-azabicyclo[2.2.1]heptan verestert oder verethert werden, wie das nachfolgende Schema veranschaulicht.

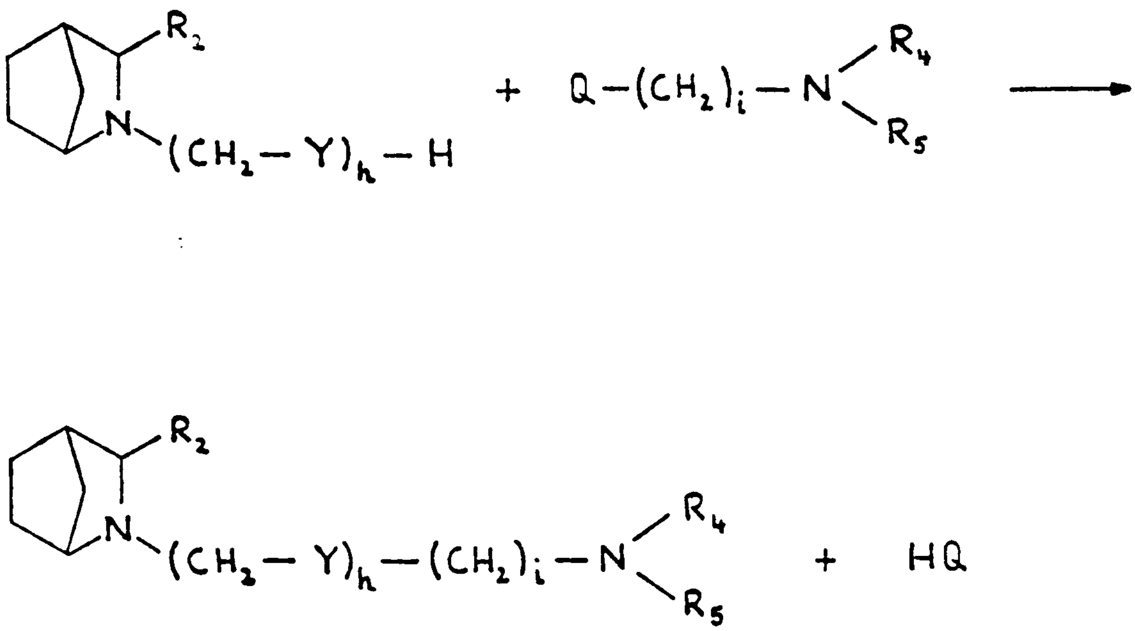

h, i = gleich oder verschieden; 2 oder 3,
$R_2$ = wasserstoff oder Alkylgruppen mit 1 bis 4 Kohlenstoffatomen, bevorzugt wasserstoff,
$R_4$, $R_5$ = gleich oder verschieden, bzw. Alkylgruppen mit 1 bis 4 C-Atomen bevorzugt Methyl,
Y = O, eine oder mehrere Alkoxygruppen mit 2-4 C-Atomen, NH oder N-Alkyl, insbesondere N-Methyl
Q = Halogen (bevorzugt Chlor), Pseudohalogen, Sulfat oder Sulfonat.

Durch die Wahl verschiedener Amine und durch Abwandlung funktioneller Gruppen in der Seitenkette ist es möglich, die Eigenschaften der 2-Azabicyclo[2.2.1]heptan-Derivate dem beabsichtigten Verwendungszweck anzupassen.

Das erfindungsgemäße Verfahren zur Herstellung dieser 2-Azabicyclo[2.2.1]heptan-Derivate erlaubt die Synthese einer Vielfalt bisher unbekannter Stoffe, die chemisch betrachtet der Klasse der tertiären Amine zugerechnet werden.

Wie dem Fachmann vertraut ist, finden tertiäre Amine vielseitige Anwendung. Ein technisch wie wirtschaftlich besonders wichtiger Anwendungsbereich der tertiären Amine ist die Kunststoffindustrie.

Hier werden tertiäre Amine als Reaktionsbeschleuniger für Vernetzungsreaktionen eingesetzt. In diesem Sinne stellt die vorliegende Erfindung im besonderen für die Polyurethan-Industrie neuartige Katalysatortypen bereit, die es erlauben, stetig neue Anforderungen wirksam begleiten zu können. Solche Anforderungen ergeben sich z. B. aus anwendungstechnischen Neuerungen, Wirtschaftlichkeitsbetrachtungen, Arbeitshygiene, Produktionssicherheit, Umweltschutz und anderem mehr.

Polyurethankunststoffe nehmen aufgrund ihrer außergewöhnlichen Anpassungsfähigkeit an anwendungstechnische Bedürfnisse heute einen breiten Raum in der industriellen Fertigung ein. Die Mannigfaltigkeit der Anwendungsmöglichkeiten hat es andererseits auch bewirkt, daß, den jeweiligen Zwecken angepaßt, die unterschiedlichsten Fertigungstechniken in den vergangenen Jahrzehnten entwikkelt wurden.

Unabhängig von der erzielten Struktur, wie z. B. Schaumstoffen, Mikrocellulosen, Elastomeren, Klebern, Lacken usw., erfolgt die Bildung eines Polyurethankörpers aus Polyolen und mehrfunktionellen Isocyanaten. Stand der Technik ist ferner, daß die unterschiedlichsten Hilfsstoffe wie z. B. Schaumstabilisatoren, Flammschutzmittel, Emulgatoren usw. dem jeweiligen Anwendungsfall entsprechend mitverwendet werden.

Eine wichtige Rolle unter den Hilfsstoffen nehmen die Katalysatoren ein. Einmal ist es ihre Aufgabe, die Bildung der Urethanbindung zu beschleunigen:

$$R_1\text{-OH} + \text{O=C=N-}R_2 \longrightarrow R_1\text{-O-}\overset{\overset{\text{O}}{\|}}{\text{C}}\text{-NH-}R_2,$$

andererseits wird bei geschäumten Systemen, sofern "wassergetriebene Schäume" betrachtet werden, auch die Umsetzung von Wasser und Isocyanat katalysiert. Bei dieser Reaktion wird Kohlendioxid als Schäumgas in situ entwickelt:

$$H_2O + O=C=N\text{-}R \rightarrow CO_2 + H_2N\text{-}R$$

Es ist Stand der Technik, daß metallorganische Verbindungen der unterschiedlichsten chemischen Strukturen und tertiäre Amine als Katalysatoren eingesetzt werden. Unter den metallorganischen Verbindungen nehmen Zinnverbindungen (wie z. B. Dibutyl-zinn(IV)dilaurat, Zinn(II)dioctoat als bekannteste Stoffe) eine besonders wichtige Stellung ein.

Während die metallorganischen Verbindungen samt und sonders Lewis-Säuren sind, zeichnen sich tertiäre Amine als Lewis-Basen ebenfalls durch starke katalytische Effekte aus.

Dem Fachmann ist geläufig, daß die gemeinsame Verwendung von Lewis-Säuren und Lewis-Basen üblicherweise durch synergistische Effekte gekennzeichnet ist. Dennoch hängt es stark vom jeweiligen Anwendungszweck ab, in welchem Umfang solche synergistisch wirkenden Systeme eingesetzt werden.

Stand der Technik ist auch, daß in einer Vielzahl von Anwendungen nur Amine zur Katalyse eingesetzt werden.

Die Zahl der Amine, die als Katalysatoren vorgeschlagen wurden, ist äußerst vielfältig, wie die einschlägige Fachliteratur aufzeigt (vgl. Stanford Res. Report No. 10 A und 10 B; Ullmann, 4. Aufl.; Kunststoffhandbuch Band VII, Polyurethane, Hanser-Verlag, München, Wien 1983).

Dennoch haben bisher nur relativ wenige Aminstrukturen auf besonders breiter Basis Eingang in die Technik gefunden. Wie dem Fachmann vertraut ist, sind dies z. B. 1,4-Diazabicyclo[2.2.2]octan, Bis-(2-dimethylaminoethyl)ether, Dimethylcyclohexylamin, Dimethylethanolamin, Dimethylbenzylamin, Methylmorpholin, Ethylmorpholin, um die wichtigsten zu nennen.

Für die Praxis hat es sich als günstig erwiesen, oft mit Amingemischen zu operieren. In diesem Sinne werden auch andere, weniger geläufige Amine wie z. B. Piperazinderivate, alkylierte Fettamine und viele andere empfohlen, wie der Fachmann weiß.

In der Technik haben sich von den vorgenannten Katalysatortypen jene besonders durchgesetzt, die eine universelle Verwendbarkeit sowie hohe Aktivität kennzeichnen und die außerdem technisch wirtschaftlich herstellbar sind.

Überraschenderweise wurde gefunden, daß bestimmte 2-Azabicyclo[2.2.1]heptan-Derivate in diesem Sinne besonders geeignet sind.

Diese 2-Azabicyclo[2.2.1]heptan-Derivate sind aus wohlfeilen Rohstoffen technisch herstellbar.

Besonders überraschend ist, daß 2-Azabicyclo[2.2.1]heptan-Katalysatoren bei gleicher C-Zahl eine erheblich höhere Aktivität besitzen als konventionelle monocyclische Aminkatalysatoren. So zeigt z. B. N-Ethyl-2-azabicyclo[2.2.1]heptan in anwendungstechnischen Formulierungen gegenüber N,N-Dimethylcyclohexylamin doppelte Wirksamkeit.

Ein anderer willkommener Effekt ist bei den erfindungsgemäß vorgeschlagenen Katalysatoren darin zu sehen, daß sich durch die Wahl geeigneter Substituenten an Stickstoff die Aktivität der Produkte - im Gegensatz zum 1,4-Diazabicyclo[2.2.2]octan, das unter wirtschaftlich vertretbaren Aspekten keine Variation erlaubt -, maßgeschneidert einstellen läßt.

So bewirkt das N-Methyl-2-azabicyclo[2.2.1]heptan eine Aktivitätssteigerung um das Drei- bis Vierfache gegenüber Dimethylcyclohexylamin, während das n-Butyl-Derivat nahezu gleiche Aktivität aufweist, zusätzlich aber den Vorteil günstigerer Verlaufseigenschaften etwa bei der Hartschaumherstellung bietet.

Die Möglichkeiten der erfindungsgemäßen Synthese erlauben es, eine Vielzahl unterschiedlichster mono- und difunktioneller primärer Amine zur Synthese der erfingungsgemäßen Katalysatoren zu verwenden.

Durch Verwendung von Alkanolaminen, wie z. B. Monoethanolamin oder 2-Aminoethoxyethanol, bei der Herstellung der erfindungsgemäßen Aminkatalysatoren, werden die Stoffe N-(2-Hydroxyethyl)-2-azabicyclo-[2.2.1]heptan und N-(2-Hydroxyethoxyethyl)-2-azabicyclo[2.2.1]heptan erzeugt.

Die Strukturtypen

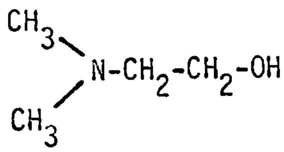

N,N-Dimethylethanolamin

und

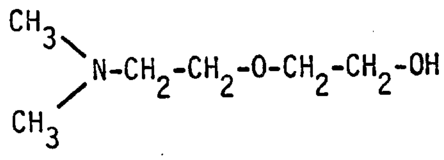

2(2-Dimethylaminoethoxy)ethanol

sind als sogenannte "einbaubare Aminkatalysatoren" im Polyurethanbereich bekannt, da die OH-Funktion ebenfalls an Isocyanat addiert werden kann.

Überraschenderweise übertreffen die aus Alkanolaminen hergestellten 2-Azabicyclo[2.2.1]heptan-Derivate die lange bekannten Stammkörper N,N-Dimethylaminoethanol und 2-(N,N-Dimethylaminoethoxy)ethanol in ihrer katalytischen Aktivität.

Zusätzlich können weitere Vorteile beobachtet werden, z. B. deutlich geringere Flüchtigkeit und damit auch deutlich verminderte Geruchsbelästigung bei der Herstellung von Polyurethan-Produkten, guter Härtungsverlauf und anderes.

Andere erfindungsgemäße N-substituierte 2-Azabicyclo[2.2.1]heptan-Derivate leiten sich von Diaminen her, deren Stammkörper z. B. sein können:

$H_2N-(CH_2)_n-NH_2$

n = 2, 3, 4, usw. (Ethylendiamin; 1,3-Diaminopropen usw.)
aber auch

$H_2N-CH_2-CH_2-O-CH_2-CH_2-NH_2$

(Bisaminoethylether)
Nach Wahl der Synthesebedingungen lassen sich bezüglich des 2-Azabicyclo[2.2.1]heptylrestes symmetrische und asymmetrische Derivate der erfindungsgemäß beanspruchten Aminkatalysatoren herstellen, wie z. B.

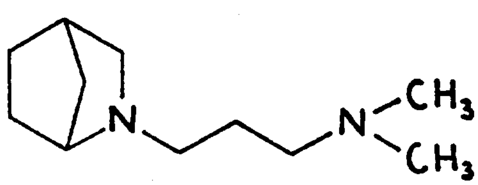

N-(3-Dimethylaminopropyl-)2-azabicyclo[2.2.1]heptan

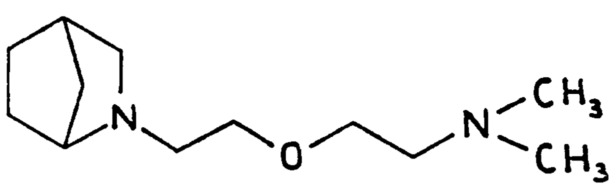

N-(2-Dimethylaminoethoxyethyl-)2-azabicyclo[2.2.1]heptan
oder

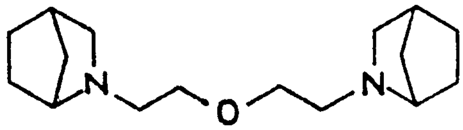

Bis-(2-azabicyclo[2.2.1]heptyl-ethyl-)ether

Alle diese Verbindungen sind durch sehr überraschende Effekte gekennzeichnet. Beispielsweise nimmt die Verbindung

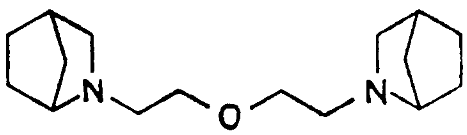

Bis-(2-azabicyclo[2.2.1]heptyl-ethyl)ether

in ihrer Aktivität, verglichen mit Bis-(2-morpholinoethyl-)ether, so außergewöhnlich zu, daß es möglich ist, mit diesem Stoff Kaltformschaum (HR-Schaum) herzustellen, wobei ein besonders rascher Härtungsverlauf erzielt wird.

Hingegen stellt Bis-(2-morpholinoethyl-)ether

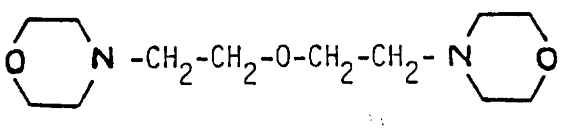

aufgrund seines Molekulargewichts und der cyclischen Substituenten am Stickstoff im Vergleich zu Bis-(2-azabicyclo[2.2.1]heptyl-ethyl-)ether, einen Katalysatortyp mit völlig anderem Erscheinungsbild dar. Bis-(2-morpholinoethyl-)ether besitzt zwar eine gute Aktivität zur Beschleunigung der Wasser/Isocyanat-Reaktion (Treibreaktion), aber nur eine geringe Gelierwirkung.

Bis-(2-morpholinoethyl-)ether ist somit zwar ein guter Treibkatalysator und wohl geeignet für Polyesterweichschaum-Applikationen, aber aufgrund der Gelieraktivität völlig ungeeignet zur Herstellung von Polyether-Weichschaum (Kaltformschaum, HR-Schaum).

Schließlich sei gesagt, daß auch die 2-Azabicyclo[2.2.1]hept-5-ene katalytische Aktivitäten im Sinne dieser Erfindung besitzen. Nachteilig ist jedoch, daß diese Stoffe sehr empfindlich sind und durch Einflüsse von Wärme, Licht und/oder Luft (Sauerstoff) sehr rasch verfärben und schließlich zersetzt werden, was ihre praktische Verwendbarkeit drastisch einschränkt.

Die Wirkungsweise der erfindungsgemäß vorgeschlagenen Polyurethankatalysatoren sei an folgenden Beispielen demonstriert:

## Beispiel 1 (Vergleichskatalysatoren)

Zur Herstellung von Polyurethan-Hartschaum werden üblicherweise Katalysatoren wie z. B. N,N-Dimethylcyclohexylamin und auch Mischungen aus 1,4-Diazabicyclo[2.2.2]octan und N,N-Dimethylaminoethanol eingesetzt. Dem Fachmann ist bekannt, daß N,N-Dimethylcyclohexylamin weltweit ein besonders dominanter Katalysator ist.

Um den Stand der Technik zu demonstrieren, wurden für eine typische Hartschaumformulierung N,N-Dimethylcyclohexylamin und eine Mischung aus 1,4-Diazabicyclo[2.2.2]octanund N,N-Dimethylaminoethanol als Vergleichskatalysatoren verwendet.

Zur Herstellung eines Hartschaumes wurde im Labor folgende Rezeptur verwendet:

Tabelle 1

| | | | |
|---|---|---|---|
| Voranol(R) 490 [1] | 33,13 g | 33,13 g | |
| Silikon DC(R) 193 [2] | 0,6 g | 0,6 g | |
| Wasser | 0,4 g | 0,4 g | |
| Trichlorfluormethan | 16,3 g | 16,30 g | |
| Rubinate(R) M [3] | 49,57 g | 49,57 g | |
| N,N-Dimethylcyclohexylamin (Vergleichskatalysator I) | 0,9 g | - | |
| 1,4-Diazabicyclo[2.2.2]octan | - | 0,16 g | 0,8 g |
| N,N-Dimethylaminoethanol (Vergleichskatalysator II) | - | 0,64 g | |
| Cremezeit (s) | 18 | 20 | |
| Gelzeit (s) | 109 | 97 | |
| Klebfreizeit (s) | 135 | 125 | |
| Steigzeit (s) | 157 | 150 | |
| Abrieb (friability) | kein | kein | |
| L-Form-Test, % (Füllung) | 82 | 79 | |

1) Produkt der Dow Chem., Neutralpolyol, OH-Zahl 490
2) Produkt der Dow Corning
3) Produkt von Rubicon polymeres MDI, Funktionalität 2.7

Beispiel 2

Um die Eigenschaften der erfindungsgemäßen neuartigen Aminkatalysatoren in Polyurethanhartschaumanwendungen zu demonstrieren, wurde die gleiche Rezeptur von Beispiel 1 verwendet, jedoch mit dem Unterschied, daß als Katalysatoren verschiedene 2-Azabicyclo[2.2.1]heptan-Derivate eingesetzt wurden. Die jeweiligen Aminkonzentrationen wurden so gewählt, daß möglichst die in Beispiel 1 angegebenen Schaumsteigzeiten eingehalten werden:

Tabelle 2

| Aminkatalysator (variiert) | Menge g | Cremezeit s | Gelzeit s | Klebfrei-s | Steigzeit s | Abrieb | L-Form-Test % Füllung |
|---|---|---|---|---|---|---|---|
| Vergleichskatalysator I | 0,9 | 18 | 109 | 135 | 157 | keine | 82 |
| Vergleichskatalysator II | 0,8 | 20 | 97 | 125 | 150 | keine | 79 |
| N-Methyl-R | 0,3 | 19 | 86 | 107 | 145 | keine | 82 |
| N-Ethyl-R | 0,5 | 16 | 107 | 134 | 151 | keine | 87 |
| N-Isopropyl-R | 1,5 | 13 | 116 | 129 | 154 | schlecht | 88 |
| N-Butyl-R | 0,8 | 16 | 93 | 109 | 147 | mäßig | 87 |
| N-Cyclohexyl-R | 2,0 | 14 | 122 | 112 | 170 | schlecht | 87 |
| N-Methoxypropyl-R | 1,0 | 16 | 109 | 118 | 155 | mäßig | 79 |
| R = 2-Azabicyclo[2.2.1]heptan | | | | | | | |

Wie aus dem Vergleich der Ergebnisse in Tabelle 2 zu ersehen ist, zeigen die erfindungsgemäß beanspruchten Aminkatalysatoren überraschende Ergebnisse gegenüber konventionellen Produkten.

Ein Vergleich der Katalysatoren N,N-Dimethylcyclohexylamin (Tabelle 2, Zeile 1, Beispiel 2) und N-Ethyl-2-azabicyclo[2.2.1]heptan (Tabelle 2, Zeile 4, Beispiel 2) zeigt, daß, obwohl beide Stoffe die gleiche Anzahl Kohlenstoffatome enthalten, N-Ethyl-2-azabicyclo[2.2.1]heptan aber nahezu die doppelte Aktivität besitzt und zusätzlich durch bessere Verlaufseigenschaften bei der Schaumbildung gekennzeichnet ist.

Die Ausfüllung einer L-Form in % des Volumens bei gleicher Aufgabemenge der Rezeptur ist ein Maß für die Fließfähigkeit des Schaums. Je höher der Formfüllungsgrad in % ist, um so bessere Fließ- und Verlaufseigenschaften erlaubt der Katalysator in einer sonst gleichen Rezeptur zu erzielen.

Beispiel 3

Dieses Beispiel illustriert, daß die erfindungsgemäßen Aminkatalysatoren im Sprühschaum angewendet, ebenfalls sehr überraschende Effekte hervorbringen.

Die Formulierung 1 stellt eine Sprühschaumrezeptur dar, wie sie auch praktisch angewendet wird unter Benutzung von N,N-Dimethylcyclohexylamin als Aminkatalysator.

Die Formulierung 2 zeigt die gleiche Sprühschaumrezeptur unter Verwendung von N-Ethyl-2-azabicyclo-[2.2.1]heptan.

Tabelle 3

| | Gewichtsteile der Rezeptur in Formulierung 1 | 2 |
|---|---|---|
| Thanol R 350 X[1)] | 16,01 | 16,13 |
| Thanol R 470 X[2)] | 6,40 | 6,45 |
| Thanol SF 265 X[3)] | 1,60 | 1,61 |
| Terate 203[4)] | 8,00 | 8,07 |
| Glycerin | 1,5 | 1,5 |
| Antiblaze 80[5)] | 4,0 | 4,0 |
| DABCO LK 443[6)] | 0,45 | 0,45 |
| Blei-octoat (24 %ig) | 0,035 | 0,035 |
| Trifluorchlormethan | 11,5 | 11,5 |
| Rubinate M[7)] | 49,5 | 50,0 |
| NCO-Index | 118 | 117 |
| Amin variiert | | |
| N,N-Dimethylcyclohexylamin | 0,5 | - |
| N-Ethyl-2-azabicyclo[2.2.1]heptan | - | 0,25 |

Fortsetzung der Tabelle 3

1) Aminbasisches Polyol, OH-Zahl 530, Produkt der Texaco Inc.
2) Aminbasisches Polyol, OH-Zahl 470, Produkt der Texaco Inc.
3) Aminbasisches Reaktivpolyol, OH-Zahl 635, Produkt der Texaco Inc.
4) Aromatisches Polyesterpolyol, Produkt von Hercules
5) Trischlorpropylphosphat, Produkt von Albright and Wilson, Inc.
6) nichtsilikonisches Surfactans, Produkt von Air Products
7) Polymer MDI, Funktionalität 2.7, Produkt von Rubicon

Die Formulierungen 1 und 2 wurden mit einem Auftragsgerät, Gusmer FF, Modell ARA (125), bei einer Kopftemperatur von 49 °C verschäumt.

Tabelle 4

| Reaktionsprofil | Formulierung 1 | Formulierung 2 |
|---|---|---|
| Cremezeit, s | 1 | 1 |
| Klebfreizeit, s | 7 | 7 |
| Steigzeit, s | 8 | 11 |
| **Physikalische Eigenschaften** | | |
| Schaumdichte, $kg/m^3$ | 40 | 43 |
| K-Faktor (btn x $inch/ft^2$ x h x F°), W/m·K | 0,0164 (0,114) | (0,115) 0,0166 |
| Abriebfestigkeit, % Gewichtsverlust | 0,28 | 0,13 |
| Wärmebiegefestigkeit, °C | 134 | 142 |
| Anteil geschlossene Zellen, % | 97,7 | 97,6 |
| Druckfestigkeit, k Pa, parallel zur Schäumrichtung | 307 | 333 |
| quer zur Schäumrichtung | 174 | 189 |

Beispiel 4

Zur Herstellung von Verpackungsschaum werden in der Technik gerne solche Aminkatalysatoren verwendet, die noch eine OH-Funktion im Molekül tragen. Dadurch wird erreicht, daß das Amin im Schaum fixiert wird und keine unerwünschten Einwirkungen auf das Verpackungsgut ausüben kann.

Ein in der Praxis verbreiteter Katalysatortyp stellt für diesen Anwendungszweck z. B. N,N-Dimethylaminoethoxyethanol dar. Gegenüber N,N-Dimethylaminoethoxyethanol und N,N-Dimethylaminoethanol als Vergleichskatalysatoren zeigen die Stoffe N-(2-Hydroxyethoxyethyl)-2-azabicyclo[2.2.1]heptan und N-Hydroxyethyl-2-azabicyclo[2.2.1]heptan folgende Reaktivitätswerte:

Tabelle 5

| | Gewichtsteile der Rezeptur in Formulierung 1 | 2 | 3 | 4 |
|---|---|---|---|---|
| Thanol SF 5505 1) | 45 | 45 | 45 | 45 |
| Thanol R 650 X 2) | 45 | 45 | 45 | 45 |
| Surfonic N 120 3) | 10 | 10 | 10 | 10 |
| Wasser | 40 | 40 | 40 | 40 |
| Silikon L 550 4) | 3,0 | 3,0 | 3,0 | 3,0 |
| Rubinate M 5) | 200 | 200 | 200 | 200 |
| NCO-Index | 31 | 31 | 31 | 31 |
| Amin (variiert) | | | | |
| N-(2-Hydroxyethoxyethyl-)-2-azabicyclo[2.2.1]heptan | 2,3 | - | - | - |
| 2-(2-Dimethylaminoethoxy-)ethanol (Vergleichskatalysator) | - | 3,0 | - | - |
| N-(2-Hydroxyethyl)-2-azabicyclo[2.2.1]heptan | - | - | 4,2 | - |
| N,N-Dimethylaminoethanol (Vergleichskatalysator) | - | - | - | 7,0 |

Fortsetzung der Tabelle 5

| | | | | |
|---|---|---|---|---|
| Cremezeit, s | 12 | 12 | 8 | 14 |
| Gelzeit, s | 28 | 31 | 28 | 30 |
| Klebfreizeit, s | 36 | 40 | 40 | 37 |
| Steigzeit, s | 35 | 38 | 38 | 37 |
| Dichte, $kg/m^3$ | 9,6 | 9,0 | - | - |

1) Polyetherpolyol, OH-Zahl 32-36 mg KOH/g, Produkt der Texaco Inc.

2) Amino-polyol, OH-Zahl 450 mg KOH/g, Produkt der Texaco Inc.

3) nichtionisches Tensid, OH-Zahl 75

4) Silikon-Tensid, Produkt von Union Carbide

5) Polymer MDI, Funktionalität 2.7, Produkt von Rubicon

Die Ergebnisse der Formulierungen 1 und 3 in Beispiel 4 zeigen, daß die darin eingesetzten 2-Azabicyclo[2.2.1]heptan-Derivate trotz ihres höheren Molgewichtes, verglichen mit den entsprechenden einfachen methylierten Alkanolaminen, in den Formulierungen 2 und 4 eingesetzt, in deutlich geringerer

Menge gleiche Leistungen erbringen.

Beispiel 5

Folienhinterfüllschaum stellt ein weiteres bedeutendes Anwendungsgebiet der Polyurethankunststoffe dar. Produkte dieser Art bestehen aus einem Schaumstoff körper (mit oder ohne Stützarmierung aus Metall, Kunststoff o. ä.) und einer dekorativen Folie, die den sichtbaren Teil des Produktes überzieht.

Üblicherweise werden PVC-Folien im Schäumungsprozeß verwendet. PVC-Folien besitzen jedoch eine große Empfindlichkeit gegenüber Aminen. Dem Fachmann ist bekannt, daß speziell solche Amine, die nicht nach Ablauf der Schäumungsreaktion im Produkt chemisch eingebaut werden (wie z. B. 1,4-Diazabicyclo-[2.2.2]octan, Trimethylaminoethylpiperazin o. ä.), zu einer besonders starken Verfärbung der Vinyldeckschicht führen. Das Interesse der Fachwelt richtet sich hier auf solche Stoffe, die der Gruppe der Alkanolamine angehören.

Einer anwendungstechnischen Optimierung von Schaumrezepturen gehen üblicherweise Versuche voraus, die im besonderen die Verfärbungstendenz jeglicher Aminkatalysatoren gegenüber PVC-Folien ermitteln sollen.

Das Beispiel 5 beschreibt einen derartigen Auswahlversuch, wobei als Aminkatalysatoren verwendet wurden:

A N-(2-Hydroxyethyl-)2-azabicyclo[2.2.1]heptan
(erfindungsgemäßer Katalysator)
B 1-N,N-Dimethylamino-3-N',N'-bis-(2-hydroxypropyl)aminopropan
(kommerzieller Vergleichskatalysator)
C N,N,N'-Trimethylaminoethylethanolamin
(kommerzieller Vergleichskatalysator)
D N,N,N'-Trimethylaminopropylethanolamin
(kommerzieller Vergleichskatalysator)
E 1-(Bis-3-dimethylaminopropyl-)aminopropan-2-ol
(kommerzieller Vergleichskatalysator)
F Dimethylaminoethanol
(kommerzieller Vergleichskatalysator)

Zunächst wurden in einem Becher Probeschäume hergestellt, um möglichst ähnliche Reaktionszeiten zu ermitteln.

Als Rezeptur wurde hierzu verwendet:

Tabelle 6

| | Gewichtsteile in Rezeptur | | | | | |
|---|---|---|---|---|---|---|
| | 1 | 2 | 3 | 4 | 5 | 6 |
| Thanol SF 3950[1)] | 97,4 | 97,4 | 97,4 | 97,4 | 97,4 | 97,4 |
| Ruß[2)] | 2,0 | 2,0 | 2,0 | 2,0 | 2,0 | 2,0 |
| Formrez II-225[3)] | 2,4 | 2,4 | 2,4 | 2,4 | 2,4 | 2,4 |
| Niax 50-970[4)] | 6,0 | 6,0 | 6,0 | 6,0 | 6,0 | 6,0 |
| Wasser | 2,2 | 2,2 | 2,2 | 2,2 | 2,2 | 2,2 |
| Rubinate M[5)] | 51,0 | 51,0 | 51,0 | 51,0 | 51,0 | 51,0 |
| Amin (variiert) | | | | | | |
| A (erfindungsgem. Katalysator) | 0,76 | - | - | - | - | - |
| B (Vergleichs-katalysator 1) | - | 1,2 | - | - | - | - |
| C (Vergleichs-katalysator 2) | - | - | 0,6 | - | - | - |
| D (Vergleichs-katalysator 3) | - | - | - | 0,64 | - | - |
| E (Vergleichs-katalysator 4) | - | - | - | - | 0,56 | - |
| F (Vergleichs-katalysator 5) | - | - | - | - | - | 0,86 |
| Steigzeit, s | 81 | 74 | 76 | 85 | 81 | 83 |
| Fadenziehzeit, s | 260 | 215 | 210 | 240 | 225 | 260 |

1) Polyether Polyol OH-Zahl 30 mg/KOH, Produkt der Texaco Inc.

2) 8 % Ruß in Polyol gelöst

3) Formrez II-225, Polyesterpolyol, Produkt von Witco

4) Niax 50-970, Polyetherpolyl, Produkt von Union Carbide Corp.

5) Polymer-MDI, Funktionalität 2.7, Produkt von Rubicon

In einer rechteckigen Form (ca. 8 x 8 cm und eine Seitenhöhe von ca. 2 cm) wurden anschließend folienbeschichtete Probekörper geschäumt. Hierzu wurde jeweils eine rote PVC-Folie (Davidson Rubber) in die Form eingelegt, anschließend geschäumt und nach 6 Minuten entformt. Aus diesen Formkörpern wurden Probestücke von 5 x 5 cm hergestellt, die nachfolgend 48 Stunden bei 121 °C (250 °F) gealtert wurden.

Eine visuelle Beurteilung der Proben hinsichtlich ihrer Verfärbung ergab:

Tabelle 7

| Katalysator | Verfärbung der Folie |
|---|---|
| A (erfindungsgemäß) | keine erkennbare Verfärbung |
| B | etwa 50 % Verfärbung |
| C | etwa 50 % Verfärbung |
| D | vollständige Verfärbung |
| E | etwa 70 - 80 % Verfärbung |
| F | etwa 10 - 20 % Verfärbung |

Wie das Beispiel 5 zeigt, wurden bei Verwendung des erfindungsgemäßen Katalysators N-(2-Hydroxyethyl-)2-azabicyclo[2.2.1]heptan überraschenderweise keine Verfärbungen der PVC-Folie im Gegensatz zu anderen Katalysatoren gefunden.

Beispiel 6

Das folgende Beispiel verdeutlicht den Einsatz von Bis-(2-azabicyclo[2.2.1]heptyl-ethyl-)ether im Vergleich zu Bis-(2-dimethylaminoethyl-)ether in weichelastischen Kaltformschäumen.

Tabelle 8

| | Gewichtsteile in Rezeptur | |
|---|---|---|
| | 1 | 2 |
| Thanol SF 5505[1)] | 60 | 60 |
| Niax 34-28[2)] | 40 | 40 |
| Silikon L 5309[3)] | 1,5 | 1,5 |
| Diethanolamin | 1,5 | 1,5 |
| Wasser | 3,5 | 3,5 |
| Didecylthiodibutylzinn | 0,06 | 0,06 |
| Bis-(2-dimethylaminoethyl-)ether[4)] | 0,3 | - |
| Dipropylenglykol | 0,13 | - |
| Bis-(2-azabicyclo[2.2.1]heptylethyl-)ether | - | 0,6 |
| Toluoldiisocyanat | 43,3 | 43,3 |
| NCO/OH-Index | 102 | 102 |
| Cremezeit, s | 5 | 5 |
| Steigzeit, s | 63 | 61 |

1) Thanol SF 5505, Polyetherpolyol OH-Zahl 33 mg KOH/g, Produkt der Texaco Inc.
2) Niax 34-28, Styrol/Acrylnitril-Polymerpolyol OH-Zahl 28 mg KOH/g, Produkt der Union Carbide Corp.
3) Produkt der Union Carbide Corp.
4) Produkt der Texaco Inc.

Die Untersuchung des Gelierverhaltens nach der Kugelfallmethode[1)] (% Gelanteil in Schaum in Abnängigkeit von der Zeit) zeigt folgende Werte:

1) Texaco-Methode, R.L. Rowton, J. Cell. Plast., 16(5), 287-92

Tabelle 9

| Steigzeit des Schaumes in s | % Gelanteil in Formulierung | |
|---|---|---|
| | 1 | 2 |
| 20 | 0 | 0 |
| 25 | 0 | 18 |
| 30 | 0 | 70 |
| 35 | 53 | 90 |
| 40 | 92 | 94 |
| 45 | - | 96 |
| 50 | 96 | 97 |
| 55 | 97 | 97 |
| 60 | 97 | 98 |

Wie zu erkennen ist, zeichnet sich die Formulierung 2, unter Verwendung eines erfindungsgemäßen Katalysatoramins durch einen raschen Aufbau des Polymergerüstes aus. Dieser Effekt wird in der Praxis meist dadurch erreicht, daß dem Treibkatalysator - in Formulierung 1 - Bis-(2-dimethylaminoethyl-)ether die Gelierreaktion unterstützende Katalysatoren, üblicherweise 1,4-Diazabicyclo[2.2.2]octan zugesetzt werden.

Im Laboratorium hergestellte Schäume nach Beispiel 6, Rezeptur 1, ließen einen Amin-Geruch erkennen, während nach Rezeptur 2 hergestellte Schäume keinerlei Amineigengeruch aufwiesen.

Beispiele

Allgemeine Arbeitsvorschrift zur Herstellung von 2-Azabicyclo[2.2.1]hept-5-en-Derivaten.

Variante A (ausgehend von freiem Amin)

5 Mol des Amins werden mit konzentrierter Salzsäure oder 50 %iger Schwefelsäure neutralisiert. Nach Zugabe von 5 Mol wäßriger Formalinlösung wird der pH-Wert auf 2,5 eingestellt. Die Immoniumsalzlösung wird auf 40 °C erwärmt und 5,5 Mol (363,6 g) Cyclopentadien werden so zugegeben, daß das Roaktionsgemisch ständig siedet, wobei die Reaktorinnentemperatur von anfänglich 40 °C auf etwa 45 °C am Reaktionsende ansteigt. Nach Beendigung der Cyclopentadienzugabe wird eine Stunde ohne äußere Wärmezufuhr nachgerührt.

Hierauf wird das überschüssige Cyclopentadien (z. T. als Dicyclopentadien) als Oberphase abgetrennt. Die Wasserphase, in der das 2-Azabicyclo[2.2.1]hept-en-Derivat als Salz gelöst ist, wird mit 5,25 Mol (420 g) 50 %iger Natronlauge versetzt, wobei die freien Basen abgeschieden werden. Bei gut wasserlöslichen Verbindungen, wie z. B. dem 2-Methyl-2-azabicyclo[2.2.1]hept-5-en, muß die Wasserphase mit NaOH oder KOH gesättigt werden.

Das Rohprodukt wird durch Zugabe von fester NaOH oder KOH getrocknet und im Vakuum destilliert.

Variante B (ausgehend von Ammoniumsalz)

Das Verfahren ist besonders zur Herstellung leicht wasserlöslicher 2-Azabicyclo[2.2.1]hept-5-en-Derivate geeignet.

5 Mol des Ammoniumsalzes, z. B. Chlorid, Sulfat oder Phosphat, werden mit 5 Mol wäßriger Formaldenydlösung unter kräftigem Rühren versetzt und auf einen pH-Wert von 2,5 eingestellt. Die weitere Versuchsdurchführung und Aufarbeitung erfolgt nach der unter Variante A gegebenen Vorschrift.

Beispiel 1

N-Methyl-2-azabicyclo[2.2.1]hept-5-en (I)

Nach Variante A werden 443,7 g (5 Mol) einer 35 %igen Lösung von Methylamin in Wasser umgesetzt. Aus 712 g wasserhaltigem Rohprodukt mit 67,5 % (I) erhält man nach Vakuumdestillation 468 g (I) mit einer Reinheit von 99 %, entsprechend einer Ausbeute von 83 % der Theorie.

Beispiel 2

N-Methyl-2-azabicyclo[2.2.1]hept-en ((I)

337,6 g Methylammoniumchlorid werden nach Variante B umgesetzt. Nach Aufarbeitung und Vakuumdestillation werden 494 g (I) mit einer Reinheit von 98,5 % erhalten; dies entspricht 87,5 % der Theorie.

Beispiel 3

N-Ethyl-2-azabicyclo[2.2.1]hept-5-en (II)

Nach Variante A werden 322 g (5 Mol) einer 70 %igen Lösung von Ethylamin in Wasser umgesetzt. Nach Aufarbeitung und Vakuumdestillation werden 575 g (II) mit einer Reinheit von 99 % erhalten; dies entspricht einer Ausbeute von 91 % der Theorie.

Beispiel 4

N-n-Propyl-2-azabicyclo[2.2.1]hept-5-en (III)

Durch Umsetzung von 296 g (5 Mol) n-Propylamin nah Variante A erhält man 570 g eines Produktes mit einem Gehalt von 98 % (III), entsprechend 80 % der Theorie.

Beispiel 5

N-i-Propyl-2-azabicyclo[2.2.1]hept-5-en (IV)

296 g (5 Mol) Isopropylamin werden nach Variante A umgesetzt. Durch Vakuumdestillation erhält man 626 g (IV) mit einer Reinheit von 97 % (IV), entsprechend einer Ausbeute von 87 % der Theorie.

Beispiel 6

N-Allyl-2-azabicyclo[2.2.1]hept-5-en (V)

Nach Variante A werden 144 g (2,5 Mol) Allylamin mit Salzsäure neutralisiert und mit 187,5 g (2,5 Mol) 40 %iger Formalinlosung und 198 g (3,0 Mol) Cyclopentadien umgesetzt. Nach Aufarbeitung und Vakuumdestillation werden 313 g eines Produktes mit einem Gehalt von 98 % (V) erhalten; dies entspricht einer Ausbeute von 91 % der Theorie.

Beispiel 7

N-Butyl-2-azabicyclo[2.2.1]hept-5-en (VI)

Durch Umsetzung von 365,7 g (5 Mol) n-Butylamin nach Variante A erhält man nach Aufarbeitung 686 g Produkt mit einem Gehalt von 98 % (VI), entsprechend einer Ausbeute von 89 % der Theorie.

Allgemeine Arbeitsvorschrift zur Herstellung von 2-Azabicyclo[2.2.1]heptan-Derivaten.

Variante C

Die Umsetzung von 5 Mol Amin mit Formal in und Cyclopentadien erfolgt nach Variante A. Zur Herabsetzung des Wasser- und Salzgehaltes im Rohprodukt wird wie folgt aufgearbeitet:
Das von nicht umgesetzten Cyclopentadien befreite saure Reaktionsprodukt wird mit 250 g Cyclohexan und 5,25 Mol (420 g) 50 %iger Natronlauge versetzt. Die Phasentrennung wird bei etwa 40 °C vorgenommen, wobei die obere Phase nach Zusatz von 2,5 g eines handelsüblichen Palladium-Trägerkatalysators mit 5 % Pd (z. B. 5 % Pd auf Kohle) unter erhöhtem Druck hydriert wird. Nach der Hydrierung wird der Katalysator abfiltriert. Nach azeotroper Trocknung des Hydrierungsproduktes, wobei das Cyclohexan als Wasserschlepper dient, wird das 2-Aza-bicyclo[2.2.1]heptan durch Destillation gewonnen.

Variante D

Gut wasserlösliche, hochsiedende Amine werden nach Variante A umgesetzt. Nach Abtrennung des nichtumgesetzten Cyclopentadiens wird das saure Reaktionsgemisch mit 200 g n-Butanol und 5,25 Mol (420 g) 50 %iger Natronlauge versetzt. Phasentrennung und Hydrierung werden wie unter Variante C beschrieben durchgeführt. Das Hydrierungsprodukt wird azeotrop unter Ausnutzung des n-Butanol/Wasser-Azeotrops getrocknet und anschließend im Vakuum destilliert.

Beispiel 8

N-Methyl-2-azabicyclo[2.2.1]heptan (VII)

Die Herstellung von (VII) erfolgt ausgehend von 388 g (5 Mol) einer 40 %igen Lösung von Methylamin in Wasser nach Variante C. Durch Destillation bei Atmosphärendruck erhält man 406 g Produkt mit 99 % (VII), entsprechend einer Ausbeute von 72 % der Theorie.
Siedepunkt: 134 °C/1013 hPa (mbar); $n_D20°$: 1,4645

Beispiel 9

N-Ethyl-2-azabicyclo[2.2.1]heptan (VIII)

Die Umsetzung von 322 g (5 Mol) einer 70 %igen Lösung von Ethylamin in Wasser wird nach Variante C durchgeführt. Nach Vakuumdestillation erhält man 463 g eines Produktes mit 98,5 % (VIII), entsprechend einer Ausbeute von 73 % der Theorie.
Siedepunkt: 64 - 65 °C/27 hPa (mbar); $n_D20°$: 1,4636

Beispiel 10

N-(2-Hydroxyethyl-)2-azabicyclo[2.2.1]heptan (IX)

Nach Variante D werden 306 g (5 Mol) Monoethanolamin umgesetzt. Durch Vakuumdestillation werden 562 g eines viskosen Produktes mit einem Gehalt von 98 % (IX) erhalten, entsprechend einer Ausbeute von 78 % der Theorie.

Beispiel 11

N-Propyl-2-azabicyclo[2.2.1]heptan (X)

296 g (5 Mol) n-Propylamin werden nach Variante C umgesetzt. Nach Vakuumdestillation verbleiben 575 g mit einem Gehalt von 98 % (X), entsprechend einer Ausbeute von 81 % der Theorie.

Beispiel 12

N-i-Propyl-2-azabicyclo[2.2.1]heptan (XI)

Nach Variante C werden 296 g (5 Mol) Isopropylamin umgesetzt. Nach Vakuumdestillation werden 580 g mit einem Gehalt von 99 % (XI) erhalten; dies entspricht einer Ausbeute von 82 % der Theorie.
Siedepunkt: 71,5 °C/25 hPa (mbar); $n_D20°$: 1,4651

Beispiel 13

N-(3-Methoxypropyl-)2-azabicyclo[2.2.1]heptan (XII)

445 g (5 Mol) 3-Methoxypropylamin werden nach Variante C umgesetzt. Das Destillat wiegt 624 g und enthält 99 % (XII), entsprechend einer Ausbeute von 73 % der Theorie.

Beispiel 14

N-n-Butyl-2-azabicyclo[2.2.1]heptan (XIII)

Nach Variante C werden 366 g (5 Mol) n-Butylamin umgesetzt. Nach der Vakuumdestillation erhält man 700 g mit einem Gehalt von 98,5 % (XIII), entsprechend einer Ausbeute von 90 % der Theorie.

Beispiel 15

N-(2-Ethylhexyl-)2-azabicyclo[2.2.1]heptan (XIV)

645 g (5 Mol) 2-Ethylhexylamin werden nach Variante C umgesetzt. Nach der Vakuumdestillation erhält man 897 g mit einem Gehalt von 98 % (XIV), entsprechend einer Ausbeute von 84 % der Theorie.

Beispiel 16

N-(2-Hydroxyethoxyethyl-)2-azabicyclo[2.2.1]heptan (XV)

Die Umsetzung von 525 g (5 Mol) Diglykolamin erfolgt nach Variante D. Nach Vakuumdestillation werden 646 g einer viskosen Flüssigkeit mit einem Gehalt von 97,5 % (XV) isoliert; dies entspricht einer Ausbeute von 68 % der Theorie.

Beispiel 17

N-Cyclohexyl-2-azabicyclo[2.2.1]heptan (XVI)

496 g (5 Mol) Cyclohexylamin werden nach Variante C umgesetzt. Nach der Vakuumdestillation werden 760 g mit einem Gehalt von 99 % (XVI) erhalten; dies entspricht einer Ausbeute von 84 % der Theorie.

Beispiel 18

N-Benzyl-2-azabicyclo[2.2.1]heptan (XVII)

Die Umsetzung von 536 g (5 Mol) Benzylamin erfolgt nach Variante C. Nach der Vakuumdestillation erhält man 846 g Produkt mit einer Reinheit von 98,5 % (XVII), entsprechend einer Ausbeute von 89 % der Theorie.

Beispiel 19

Bis-(2-azabicyclo[2.2.1]heptyl-)ethylether (XVIII)

520 g (5 Mol) Bisaminodiethylether werden nach Variante D umgesetzt. Nach der Vakuumdestillation verbleiben 1200 g Produkt mit 98 % (XVIII), entsprechend einer Ausbeute von 89 % der Theorie.

Beispiel 20

N-(2-Hydroxypropyl)2-azabicyclo[2.2.1]heptan (XIX)

Die Umsetzung von 950 g (12 Mol) 1-Amino-2-propanol erfolgt nach Variante D. Nach der Vakuumdestillation erhält man 817 g einer viskosen Flüssigkeit mit einem Gehalt von 96,5 % (XIX); dies entspricht einer Ausbeute von 50 % der Theorie.

Beispiel 21

N-(3-Dimethylaminopropyl-)2-azabicyclo[2.2.1]heptan (XX)

511 g (5 Mol) Dimethylaminopropylamin werden nach Variante D umgesetzt. Nach der Vakuumdestillation wurden 103 g einer leicht gelben Flüssigkeit mit einem Gehalt von 97 % (XX) erhalten, entsprechend

einer Ausbeute von 11 % der Theorie; aus dem Rückstand läßt sich eine zweite 187 g wiegende Fraktion vom Siedepunkt 142 - 146 °C/25 hPa (mbar), gaschromatographisch lassen sich drei Komponenten nachweisen.

Abwandlung von 2-Azabicyclo[2.2.1]heptan-Derivaten

Veresterung

Beispiel 22

N-(2-Acetoxyethoxyethyl-)2-azabicyclo[2.2.1]heptan (XXI)

286 g (1,55 Mol) N-(2-Hydroxyethoxyethyl-)2-azabicyclo[2.2.1]heptan (XV) werden vorgelegt und unter Feuchtigkeitsausschluß so mit 198 g (1,94 Mol) Essigsäureanhydrid versetzt, daß die Temperatur zwischen 50 und 60 °C gehalten wird. Hierauf wird drei Stunden zum Sieden erhitzt. Nach dem Abkühlen werden 200 ml Ether zugesetzt; das Produkt wird mit Sodalösung neutralisiert, neutral gewaschen und nach Abziehen des Ethers im Hochvakuum destilliert. Der Hauptlauf wiegt 185 g und enthält 99 % (XXI), entsprechend einer Ausbeute von 52 % der Theorie.

Veretherung

Beispiel 23

N-(2-Dimethylaminoethoxyethyl-)2-azabicyclo[2.2.1]heptan (XXII)

384 g (2,7 Mol) N-(2-Hydroxyethyl-)2-azabicyclo[2.2.1]heptan (IX) werden mit 80 g (2,0 Mol) NaOH versetzt und unter Rühren auf 105 °C aufgeheizt. Innerhalb von 30 Minuten wird eine frisch bereitete Lösung von 1-Chlor-2-diemthylaminoethan in ca. 250 ml Toluol (ausgehend von 288 g (2,0 Mol) des Hydrochlorids und 240 g 50 %ige Natronlauge) unter kräftigem Rühren hinzugefügt. Nach sechsstündigem Erhitzen auf Rückflußtemperatur wird auf Raumtemperatur abgekühlt und vom ausgefallenen NaCl abfiltriert. Nach zweimaligem Waschen mit Toluol wird das Lösemittel abgetrieben und über eine 40 cm Füllkörperkolonne im Vakuum destilliert. Man erhält 150 g Hauptfraktion mit einem Gehalt von 99,5 % (XXII), entsprechend einer Ausbeute von 35 % der Theorie, bezogen auf eingesetztes 1-Chlor-2-dimethylaminoethan-hydrochlorid.

## Tabelle 10

### Physikalische Kenndaten der neuen Verbindungen der Formel A

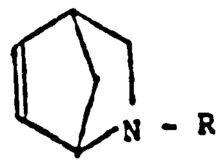

| Produkt | R | $n_D^{20°}$ | kp/T |
|---|---|---|---|
| I | $CH_3$* | 1,4744 | 67 kPa (mbar)/49°C |
| II | $CH_2-CH_3$ | 1,4728 | 20 kPa (mbar)/50°C |
| III | $CH_2-CH_2-CH_3$ | 1,4720 | 27 kPa (mbar)/70°C |
| IV | 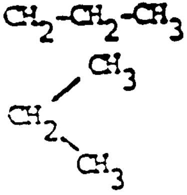 | 1,4724 | 20 kPa (mbar)/53°C |
| V | $CH=CH-CH_3$ | 1,4869 | 20 kPa (mbar)/45°C |
| VI | $CH_2-CH_2-CH_2-CH_3$ | 1,4714 | 11 kPa (mbar)/68°C |

*Bei S.D.Larsen und P.A.Grieco, J.Amer.Chem.Soc. 107, 1768 (1985) beschrieben, jedoch ohne Angabe der physikalischen Daten.

Tabelle 11

Physikalische Kenndaten der neuen Verbindungen der Formel B

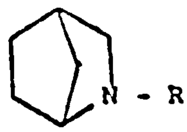

| Produkt | R | $n_D^{20°}$ | kp/T |
|---|---|---|---|
| IX | $CH_2-CH_2-OH$ | 1,4987 | 22 kPa (mbar)/109°C |
| X | $CH_2-CH_2-CH_3$ | 1,4635 | 27 kPa (mbar)/ 70°C |
| XII | $CH_2-CH_2-CH_2-OCH_3$ | 1,4674 | 20 kPa (mbar)/ 99°C |
| XIII | $CH_2-CH_2-CH_2-CH_3$ | 1,4642 | 27 kPa (mbar)/ 87°C |
| XIV | $CH_2CH(C_2H_5)-CH_2-CH_2-CH_2-CH_3$ | 1,4654 | 3 kPa (mbar)/103°C |
| XV | $CH_2-CH_2-OCH_2-CH_2-CH$ | 1,4905 | 0,1 kPa (mbar)/101-102°C |
| XVI | 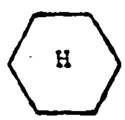 | 1,5005 | 20 kPa (mbar)/121-122°C |
| XVII | $CH_2$- 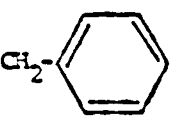 | 1,5401 | 20 kPa (mbar)/140°C |
| XVIII | $CH_2-CH_2-OCH_2-CH_2-N$ 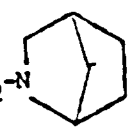 | 1,5030 | 0,9 kPa (mbar)/143°C |
| XIX | $CH_2-CH(OH)-CH_3$ | 1,4790 | 27 kPa (mbar)/102-103°C |
| XX | $CH_2-CH_2-CH_2-N(CH_3)_2$ | 1,4730 | 25 kPa (mbar)/112 °C |
| XXI | $CH_2-CH_2-O-CH_2-CH_2-O-Ac$ | 1,4770 | 0,2 kPa (mbar)/102 - 110 °C |
| XXII | $CH_2-CH_2-O-CH_2-CH_2-N(CH_3)_2$ | 1,4731 | 20 kPa (mbar)/139 °C |

## Patentansprüche

**Patentansprüche für folgende Vertragsstaaten : AT, BE, CH, DE, FR, GB, IT, LI, NL, SE**

**1.** Anwendung von in 2- und ggf. in 3-Stellung substituierten 2-Azabicyclo[2.2.1.]hept-5-en-Derivaten der allgemeinen Formel I

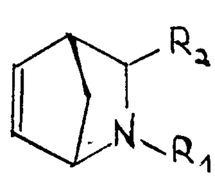

(I)

und der entsprechenden 2-Azabicyclo[2.2.1.]heptan-Derivate der allgemeinen Formel II

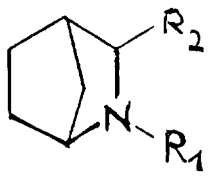

(II)

worin

R₁ → $R_1$ Alkylgruppen mit 1 bis 8 Kohlenstoffatomen, Alkenylgruppen mit 2 bis 8 Kohlenstoffatomen, Cycloalkyl- oder Alkylcycloalkylgruppen mit 5 bis 9 Kohlenstoffatomen, die Benzylgruppe, Alkylbenzylgruppen mit 8 bis 10 Kohlenstoffatomen, Heteroarylgruppen mit einem oder mehreren Heteroatomen N, O oder S mit 3 bis 10 Kohlenstoffatomen, $-(CH_2)_a$-P, $-(CH_2)_b$-T-$(CH_2)_c$-U
oder $-(CH_2)_d$-V-$(CH_2)_e$-W-$(CH_2)_f$-X
wobei a bis f = 2, 3 oder 4,

P, U, X = gleich oder verschieden sind und OH, O-Acyl, N-Acyl, N-Alkyl, N-Dialkylwobei der Alkylrest 1 bis 4 Kohlenstoffatome aufweist und vorzugsweise Methyl ist - oder 2-Azabicyclo[2.2.1]-hept-2-yl

T, V, W = gleich oder verschieden sind und O, NH oder N-Alkyl - wobei der Alkylrest 1 bis 4 Kohlenstoffatome aufweist und vorzugsweise Methyl ist - und

$R_2$ Wasserstoff oder Alkylgruppen mit 1 bis 4 Kohlenstoffatomen, bevorzugt Wasserstoff, bedeuten,

als Katalysatoren zur Herstellung von Polyurethanen und Polyepoxidharzen allein oder in Abmischung mit anderen Katalysatoren.

**2.** Herstellung von in 2- und ggf. 3-Stellung substituierten Derivaten des 2-Azabicyclo[2.2.1]hept-5-ens durch Umsetzung von Verbindungen der Formel III mit Verbindungen der Formel IV und Reaktion des so entstandenen Iminsalzes mit Cyclopentadien zu Verbindungen der Formel V,

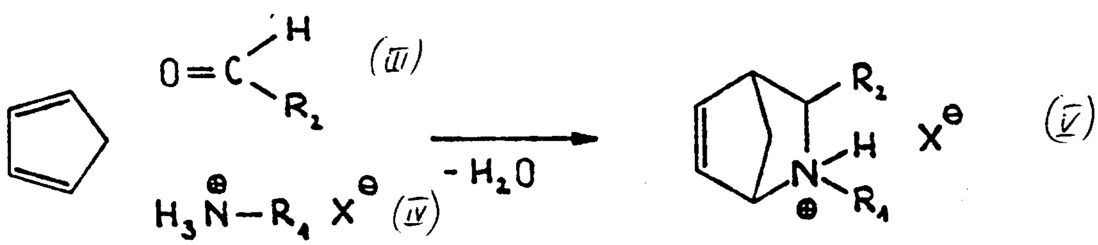


(III) (IV) (V)

worin

$R_1$ Alkyl- mit 3 bis 8 Hohlenstoffatomen in unverzweigter Kette und 5 - 8 Kohlenstoffatomen in verzweigter Kette, Alkenylgruppen mit 2 bis 8 Kohlenstoffatomen, Cycloalkyl- oder Alkylcycloalkylgruppen mit 5 bis 9 Kohlenstoffatomen, Alkylbenzylgruppen mit 8 bis 10 Kohlenstoffatomen, Heteroarylgruppen mit einem oder mehreren Heteroatomen N, O oder S mit 3 bis 10 Kohlenstoffatomen, $-(CH_2)_a$-P oder $-(CH_2)_b$-T-$(CH_2)_c$-U,
wobei a bis c = 2, 3 oder 4,

P, U = gleich oder verschieden sind und OH, O-Acyl, N-Acyl, N-Alkyl, N-Dialkyl- wobei der Alkylrest 1 bis 4 Kohlenstoffatome aufweist und vorzugsweise Methyl ist - oder 2-Azabicyclo[2.2.1]-hept-2-yl

T = O, NH oder N-Alkyl - wobei der Alkylrest 1 bis 4 Kohlenstoffatome aufweist und vorzugsweise Methyl ist - und

$R_2$ Wasserstoff oder Alkylgruppen mit 1 bis 4 Kohlenstoffatomen, bevorzugt Wasserstoff,

X der Rest einer starken Säure bedeuten,

Gewinnung der freien Basen durch Neutralisation der Salze mit mindestens äquivalenten Mengen einer starken Base oder durch Behandlung mit basischen Austauscherharzen und gegebenenfalls katalytischen Hydrierung der freien Basen in Gegenwart von Edelmetallkatalysatoren zu den entsprechenden Derivaten des 2-Azabicyclo[2.2.1]heptans.

3. Verfahren nach Anspruch 2, **dadurch gekennzeichnet,** daß Salze primärer Amine gemäß Formel IV verwendet werden, die in situ erzeugt werden.

4. Verfahren nach Anspruch 2 oder 3,
**dadurch gekennnzeichnet,**
daß als Carbonylverbindung gemäß Formel III Formaldehyd eingesetzt wird.

5. Verfahren nach einem der Ansprüche 2 bis 4,
**dadurch gekennnzeichnet,**
daß die Umsetzung in einem Zweiphasensystem in einem pH-Bereich zwischen 0 und 7, vorzugsweise zwischen pH 2 und 6, und einem Temperaturbereich zwischen 20 und 45 °C, vorzugsweise zwischen 10 und 45 °C, und insbesondere 20 bis 40 °C, durchgeführt wird.

6. Verfahren nach einem der Ansprüche 2 bis 5,
**dadurch gekennnzeichnet,**
daß Cyclopentadien im Molverhältnis 0,5 : 1 bis 2 : 1, bezogen auf das Iminiumsalz, bevorzugt im Molverhältnis 1 : 1 bis 1,5 : 1, insbesondere 1,1 : 1, eingesetzt wird.

7. Verfahren nach einem der Ansprüche 2 bis 6,
**dadurch gekennnzeichnet,**
daß die Hydrierung unter Druck in Gegenwart von Metallen der Platingruppe, insbesondere in Gegenwart von Palladium, durchgeführt wird.

8. Verfahren nach einem der Ansprüche 2 bis 7,
**dadurch gekennnzeichnet,** daß in Abänderung des Verfahrens nach Anspruch 2 die im Laufe des Verfahrens in Lösung erhaltenen Salze gemäß Formel V direkt hydriert werden.

9. Verfahren nach einem der Ansprüche 2 bis 8,
**dadurch gekennnzeichnet,**
daß die Hydrierung bei Umgebungstemperatur oder erhöhter Temperatur im Bereich zwischen 20 und 150 °C, bevorzugt zwischen 50 und 100 °C, und bei erhöhtem Druck im Bereich zwischen 1 und 15 MPa (10 und 150 bar), bevorzugt zwischen 2 und 5 MPa (20 und 50 bar), durchgeführt wird.

10. Verfahren zur Herstellung von Azabicyclo[2.2.1]heptan-Derivaten durch Umwandlung von nach einem der Ansprüche 2 bis 9 hergestellten Verbindungen, bei denen der Substituent $R_1$ eine OH- oder NH-Gruppe enthält, mit Acylierungsmitteln wie Carbonsäuren, deren Anhydride oder Halogenide gemäß der Gleichung VI

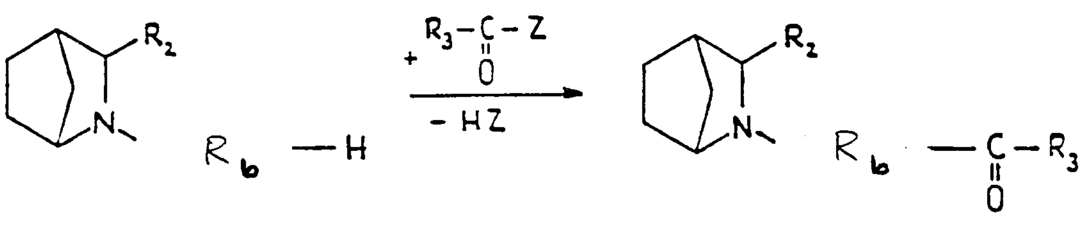


(VI)

wobei

| | |
|---|---|
| $R_6$ | $(CH_2)_a$-V oder $(CH_2)_b$-T-$(CH_2)_c$-W bedeutet, |
| | a bis c = 2, 3 oder 4, |
| V, W = | -O- oder N-Alkyl, wobei der Alkylrest 1-4 C-Atome aufweist, |
| T = | O, NH oder N-Alkyl, wobei der Alkylrest 1-4 C-Atome aufweist und verzugsweise Methyl ist und |
| $R_2$ | Wasserstoff oder eine Alkylgruppe mit 1-4 C-Atomen bedeutet. |

11. Verfahren zur Herstellung von Azabicyclo[2.2.1]heptan-Derivaten durch Umwandlung von nach einem der Ansprüche 2 bis 9 hergestellten Verbindungen, bei denen der Substituent $R_1$ eine OH-Gruppe enthält in Gegenwart starker Baden mit halogenhaltigen Verbindungen, insbesondere mit 2-, 3- oder 4-Halogenalkylaminen (insbesondere Chlorverbindungen), gemäß der Gleichung VII

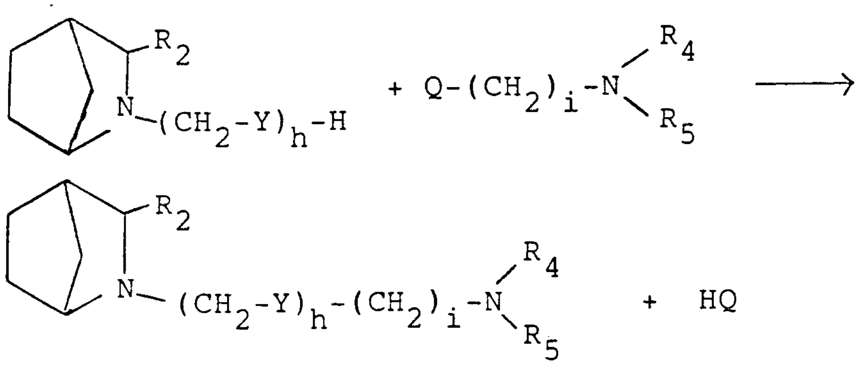

(VII)

worin

| | |
|---|---|
| h, i = | gleich oder verschieden sind und 2 oder 3, |
| Y = | Sauerstoff, eine oder mehrere Alkoxygruppen mit 2 bis 4 Kohlenstoffatomen, NH oder N-Alkyl, insbesondere N-Methyl, |
| Q = | Halogen (bevorzugt Chlor ), Pseudohalogen, Sulfat oder Sulfonat, |
| $R_2$ = | Wasserstoff oder Alkylgruppen mit 1 bis 4 Kohlenstoffatomen, bevorzugt Wasserstoff, |
| $R_4$, $R_5$ = | gleich oder verschieden sind und Alkylgruppen mit 1 bis 4 Kohlenstoffatomen, bevorzugt Methyl, |

bedeuten.

**12.** 2-Azabicyclo[2.2.1.]hept-5-en-Derivate der Formel VIII

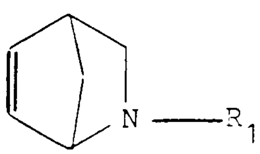 ,

(VIII)

worin $R_1$ Ethyl, Propyl, i-Propyl, Allyl oder Butyl bedeuten.

**13.** 2-Azabicyclo[2.2.1.]heptan-Derivate der Formel

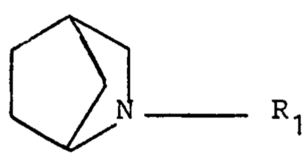 ,

worin $R_1$ die Reste 2-Hydroxyethyl, n-Propyl, 3-Methoxypropyl, n-Butyl, 2-Ethylhexyl, 2-Hydroxyethoxyethyl, Cyclohexyl, Benzyl, 3-Dimethylaminopropyl, 2-Acetoxyethoxyethyl oder 2-Dimethylaminoethoxyethyl bedeuten.

**14.** Bis-(2-azabicyclo[2.2.1.]heptyl-N-ethyl-)ether.

**Patentansprüche für folgenden Vertragsstaat : ES**

**1.** Verfahren zur Herstellung von Polyurethanen und Polyepoxidharzen, dadurch gekennzeichnet, daß man die Ausgangsmaterialien in Anwesenheit von in 2- und ggf. in 3-Stellung substituierten 2-Azabicyclo-[2.2.1]hept-5-en-Derivaten der allgemeinen Formel I

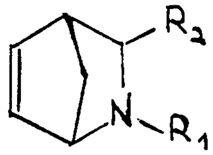

(I)

oder der entsprechenden 2-Azabicyclo[2.2.1.]heptan-Derivate der allgemeinen Formel II

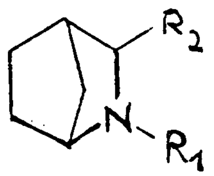

(II)

worin

$R_1$ Alkylgruppen mit 1 bis 8 Kohlenstoffatomen, Alkenylgruppen mit 2 bis 8 Kohlenstoffatomen, Cycloalkyl- oder Alkylcycloalkylgruppen mit 5 bis 9 Kohlenstoffatomen, die Benzylgruppe, Alkylbenzylgruppen mit 8 bis 10 Kohlenstoffatomen, Heteroarylgruppen mit einem oder mehreren Heteroatomen N, O oder S mit 3 bis 10 Kohlenstoffatomen, $-(CH_2)_a-P$, $-(CH_2)_b-T-(CH_2)_c-U$
oder $-(CH_2)_d-V-(CH_2)_e-W-(CH_2)_f-X$
wobei a bis f = 2, 3 oder 4,

P, U, X = gleich oder verschieden sind und OH, O-Acyl, N-Acyl, N-Alkyl, N-Dialkylwobei der Alkylrest 1 bis 4 Kohlenstoffatome aufweist und vorzugsweise Methyl ist - oder 2-Azabicyclo[2.2.1.]-hept-2-yl,

T, V, W = gleich oder verschieden sind und O, NH oder N-Alkyl - wobei der Alkylrest 1 bis 4 Kohlenstoffatome aufweist und vorzugsweise Methyl ist - und

$R_2$ Wasserstoff oder Alkylgruppen mit 1 bis 4 Kohlenstoffatomen, bevorzugt Wasserstoff, bedeuten,

als Katalysatoren allein oder in Abmischung mit anderen Katalysatoren umsetzt.

**2.** Verfahren zur Herstellung von in 2- und ggf. 3-Stellung substituierten Derivaten des 2-Azabicyclo[2.2.1]-hept-5-ens durch Umsetzung von Verbindungen der Formel III mit Verbindungen der Formel IV und Reaktion des so entstandenen Iminsalzes mit Cyclopentadien zu Verbindungen der Formel V,

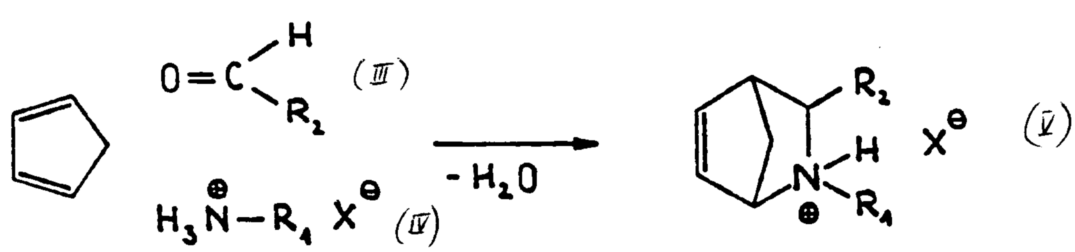


worin

$R_1$ Alkyl mit 3 bis 8 Kohlenstoffatomen in unverzweigter Kette und 5 - 8 Kohlenstoffatomen in verzweigter Kette, Alkenylgruppen mit 2 bis 8 Kohlenstoffatomen, Cycloalkyl- oder Alkylcycloalkylgruppen mit 5 bis 9 Kohlenstoffatomen, Alkylbenzylgruppen mit 8 bis 10 Kohlenstoffatomen, Heteroarylgruppen mit einem oder mehreren Heteroatomen N, O oder S mit 3 bis 10 Kohlenstoffatomen, $-(CH_2)_a-P$ oder $-(CH_2)_b-T-(CH_2)_c-U$,
wobei a bis c = 2, 3 oder 4,

P, U = gleich oder verschieden sind und OH, O-Acyl, N-Acyl, N-Alkyl, N-Dialkyl- wobei der Alkylrest 1 bis 4 Kohlenstoffatome aufweist und vorzugsweise Methyl ist - oder 2-Azabicyclo[2.2.1]-hept-2-yl

T = O, NH oder N-Alkyl - wobei der Alkylrest 1 bis 4 Kohlenstoffatome aufweist und vorzugsweise Methyl ist - und

| | |
|---|---|
| $R_2$ | Wasserstoff oder Alkylgruppen mit 1 bis 4 Kohlenstoffatomen, bevorzugt Wasserstoff, |
| X | der Rest einer starken Säure bedeuten, |

Gewinnung der freien Basen durch Neutralisation der Salze mit mindestens äquivalenten Mengen einer starken Base oder durch Behandlung mit basischen Austauscherharzen und gegebenenfalls katalytischen Hydrierung der freien Basen in Gegenwart von Edelmetallkatalysatoren zu den entsprechenden Derivaten des 2-Azabicyclo[2.2.1]heptans.

3. Verfahren nach Anspruch 2, **dadurch gekennzeichnet,** daß Salze primärer Amine gemäß Formel IV verwendet werden, die in situ erzeugt werden.

4. Verfahren nach Anspruch 2 oder 3, **dadurch gekennzeichnet,** daß als Carbonylverbindung gemäß Formel III Formaldehyd eingesetzt wird.

5. Verfahren nach einem der Ansprüche 2 bis 4, **dadurch gekennzeichnet,** daß die Umsetzung in einem Zweiphasensystem in einem pH-Bereich zwischen 0 und 7, vorzugsweise zwischen pH 2 und 6, und einem Temperaturbereich zwischen 20 und 45 °C, vorzugsweise zwischen 10 und 45 °C, und insbesondere 20 bis 40 °C, durchgeführt wird.

6. Verfahren nach einem der Ansprüche 2 bis 5, **dadurch gekennzeichnet,** daß Cyclopentadien im Molverhältnis 0,5 : 1 bis 2 : 1, bezogen auf das Iminiumsalz, bevorzugt im Molverhältnis 1 : 1 bis 1,5 : 1, insbesondere 1,1 : 1, eingesetzt wird.

7. Verfahren nach einem der Ansprüche 2 bis 6, **dadurch gekennzeichnet,** daß die Hydrierung unter Druck in Gegenwart von Metallen der Platingruppe, insbesondere in Gegenwart von Palladium, durchgeführt wird.

8. Verfahren nach einem der Ansprüche 2 bis 7, **dadurch gekennnzeichnet,** daß in Abänderung des Verfahrens nach Anspruch 2 die im Laufe des Verfahrens in Lösung erhaltenen Salze gemäß Formel V direkt hydriert werden.

9. Verfahren nach einem der Ansprüche 2 bis 8, **dadurch gekennnzeichnet,** daß die Hydrierung bei Umgebungstemperatur oder erhöhter Temperatur im Bereich zwischen 20 und 150 °C, bevorzugt zwischen 50 und 100 °C, und bei erhöhtem Druck im Bereich zwischen 1 und 15 MPa (10 und 150 bar), bevorzugt zwischen 2 und 5 MPa (20 und 50 bar), durchgeführt wird.

10. Verfahren zur Herstellung von Azabicyclo[2.2.1]heptan-Derivaten durch Umwandlung von nach einem der Ansprüche 2 bis 9 hergestellten Verbindungen, bei denen der Substituent $R_1$ eine OH- oder NH-Gruppe enthält, mit Acylierungsmitteln wie Carbonsäuren, deren Anhydride oder Halogenide gemäß der Gleichung VI

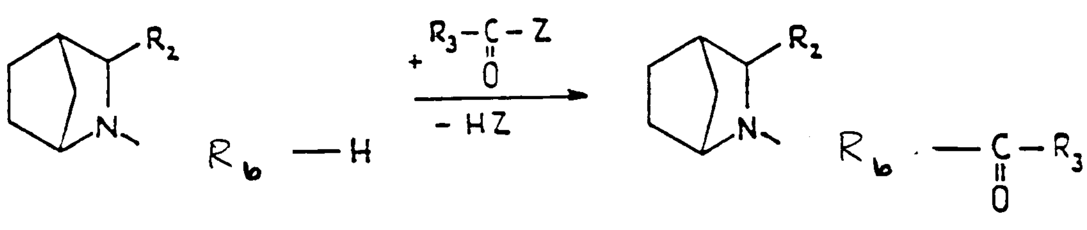


(VI)

wobei

| | |
|---|---|
| $R_6$ | $(CH_2)_a$-V oder $(CH_2)_b$-T-$(CH_2)_c$-W bedeutet, |
| | a bis c = 2, 3 oder 4, |
| V, W = | O oder N-Alkyl, wobei der Alkylrest 1-4 C-Atome aufweist |

T = O, NH oder N-Alkyl, wobei der Alkylrest 1-4 C-Atome aufweist und vorzugsweise Methyl ist und

$R_2$ Wasserstoff oder eine N-Alkylgruppe mit 1-4 C-Atomen bedeutet.

**11.** Verfahren zur Herstellung von Azabicyclo[2.2.1]heptan-Derivaten durch Umwandlung von nach einem der Ansprüche 2 bis 9 hergestellten Verbindungen, bei denen der Substituent $R_1$ eine OH-Gruppe enthält in Gegenwart starker Basen mit halogenhaltigen Verbindungen, insbesondere mit 2-, 3- oder 4-Halogenalkylaminen (insbesondere Chlorverbindungen), gemäß der Gleichung VII

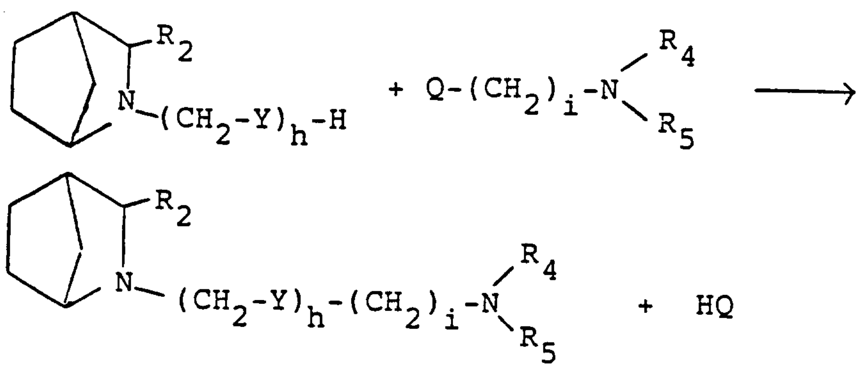

(VII)

worin

h, i = gleich oder verschieden sind und 2 oder 3,

Y = Sauerstoff, eine oder mehrere Alkoxygruppen mit 2 bis 4 Kohlenstoffatomen, NH oder N-Alkyl, insbesondere N-Methyl,

Q = Halogen ( bevorzugt Chlor ), Pseudohalogen, Sulfat oder Sulfonat,

$R_2$ = Wasserstoff oder Alkylgruppen mit 1 bis 4 Kohlenstoffatomen, bevorzugt Wasserstoff,

$R_4$, $R_5$ = gleich oder verschieden sind und Alkylgruppen mit 1 bis 4 Kohlenstoffatomen, bevorzugt Methyl,

bedeuten.

**Claims**

**Claims for the following Contracting States : AT, BE, CH, DE, FR, GB, IT, LI, NL, SE**

**1.** Use of 2-azabicyclo[2.2.1]hept-5-ene derivatives substituted in position 2 and, optionally, in position 3, of the general formula I

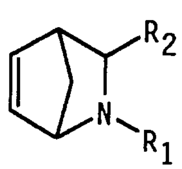

( I )

and of the corresponding 2-azabicyclo[2.2.1]heptane derivatives of the general formula II

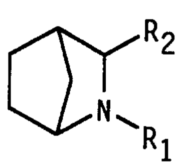

( II )

wherein:

$R_1$ represents alkyl groups with 1 to 8 carbon atoms, alkenyl groups with 2 to 8 carbon atoms, cycloalkyl or alkylcycloalkyl groups with 5 to 9 carbon atoms, the benzyl group,

alkylbenzyl groups with 8 to 10 carbon atoms, heteroaryl groups with one or more of the heteroatoms N, O, or S with 3 to 10 carbon atoms, $-(CH_2)_a-P$, $-(CH_2)_b-T-(CH_2)_c-U$, or $-(CH_2)_d-V-(CH_2)_e-W-(CH_2)_f-X$
wherein: a through f are 2, 3, or 4

P, U, X — are equal or different and are OH, O-acyl, N-acyl, N-alkyl, N-dialkyl, the alkyl residue having 1 to 4 carbon atoms and, preferably, being methyl, or 2-azabicyclo[2.2.1]-hept-2-yl,

T, V, W — are equal or different and are O, NH, or N-alkyl, the alkyl residue having 1 to 4 carbon atoms and, preferably, being methyl,

$R_2$ — represents hydrogen or alkyl groups with 1 to 4 carbon atoms, preferably hydrogen,

as catalysts, alone or as mixtures with other catalysts, for the production of polyurethanes and polyepoxy resins.

**2.** Process for the preparation of 2-azabicyclo[2.2.1]hept-5-ene derivatives substituted in position 2 or, optionally, in position 3 by reacting compounds of formula III with compounds of formula IV and by reacting the imine salt thus obtained with cyclopentadiene to yield the compounds of formula V,

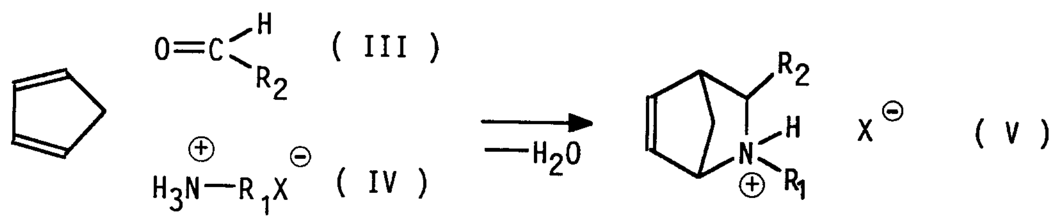

wherein:

$R_1$ — represents alkyl with 3 to 8 carbon atoms in the unbranched chain and 5 to 8 carbon atoms in the branched chain, alkenyl groups with 2 to 8 carbon atoms, cycloalkyl or alkylcycloalkyl groups with 5 to 9 carbon atoms, alkylbenzyl groups with 8 to 10 carbon atoms, heteroaryl groups with one or more of the heteroatoms N, O, or S with 3 to 10 carbon atoms, $-(CH_2)_a-P$, or $-(CH_2)_b-T-(CH_2)_c-U$,
wherein: a through c are 2, 3 or 4,

P, U — are equal or different and are OH, O-acyl, N-acyl, N-alkyl, N-dialkyl, the alkyl residue having 1 to 4 carbon atoms and, preferably, being methyl, or 2-azabicyclo[2.2.1]-hept-2-yl,

T — is O, NH, or N-alkyl, the alkyl residue having 1 to 4 carbon atoms and, preferably, being methyl

$R_2$ — represents hydrogen or alkyl groups with 1 to 4 carbon atoms, preferably hydrogen

X — represents the residue of a strong acid,

preparation of the free bases by neutralization of the salts with at least equivalent amounts of a strong base or by treatment with basic exchange resins and, optionally, catalytic hydrogenation of the free bases in the presence of noble metal catalysts to yield the corresponding 2-azabicyclo[2.2.1]heptane derivatives.

**3.** Process according to claim 2, wherein salts of primary amines according to formula IV are used, which are formed in situ.

**4.** Process according to claim 2 or 3, wherein formaldehyde is used as the carbonyl compound according to formula III.

**5.** Process according to any one of the claims 2 to 4, wherein the reaction is performed in a two-phase system in a pH range of between 0 and 7, preferably between 2 and 6, and in a temperature range of between 20 and 45 °C, preferably between 10 and 45 °C, and particularly between 20 and 40 °C.

**6.** Process according to any one of the claims 2 to 5, wherein cyclopentadiene is used at a mole ratio of 0.5 : 1 to 2 : 1, relative to the iminium salt, preferably at a mole ratio of 1 : 1 to 1.5 : 1, particularly 1.1 : 1.

**7.** Process according to any one of the claims 2 to 6, wherein the hydrogenation is performed under pressure in the presence of metals of the platinum group, particularly in the presence of palladium.

**8.** Process according to any one of the claims 2 to 7, wherein, in alteration of the process defined in claim 2, the salts according to formula V which are obtained in solution during the process are directly hydrogenated.

**9.** Process according to any one of the claims 2 to 8, wherein the hydrogenation is performed at ambient or elevated temperature of between 20 and 150 °C, preferably between 50 and 100 °C, and at elevated pressure of between 1 and 15 mPa, preferably between 2 and 5 mPa.

**10.** Process for preparing azabicyclo[2.2.1]heptane derivatives by reaction of the compounds prepared according to any one of the claims 2 to 9, the substituent $R_1$ of which containing an OH- or NH-group, with acylating agents such as carboxylic acids, the anhydrides or halogenides thereof, according to equation VI

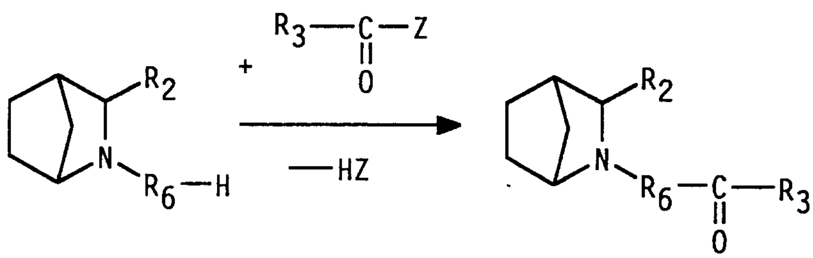

( VI )

wherein

| | |
|---|---|
| $R_6$ | is $-(CH_2)_a-V$, or $-(CH_2)_b-T-(CH_2)_c-W$, a through c being 2, 3 or 4 |
| V, W | is O or N-alkyl, the alkyl residue having 1 to 4 carbon atoms |
| T | is O, NH or N-alkyl, the alkyl residue having 1 to 4 carbon atoms and, preferably, being methyl |
| $R_2$ | is hydrogen or an alkyl group with 1 to 4 carbon atoms, |

**11.** Process for preparing azabicyclo[2.2.1]heptane derivatives by reaction of the compounds prepared according to any one of the claims 2 to 9, the substituent $R_1$ of which containing an OH group, in the presence of strong bases with halogen-containing compounds, particularly with 2-, 3-, or 4-halogen alkyl amines (particularly chlorine compounds), according to equation VII

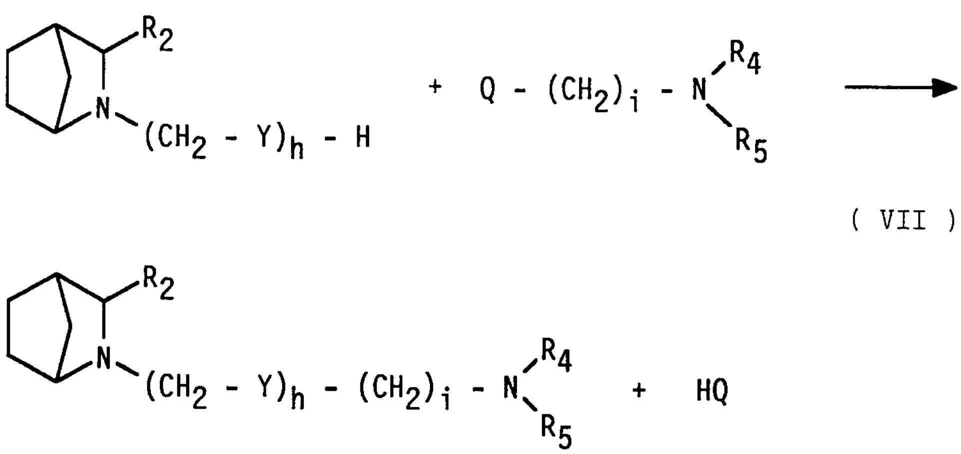

( VII )

wherein:

| | |
|---|---|
| h, i | are equal or different and are 2 or 3, |
| Y | is oxygen, one or more alkoxy groups with 2 to 4 carbon atoms, NH, or N-alkyl, particularly N-methyl |

Q is halogen, preferably chlorine, pseudohalogen, sulfate, or sulfonate

$R_2$ is hydrogen or alkyl groups with 1 to 4 carbon atoms, preferably hydrogen

$R_4$, $R_5$ are equal or different and represent alkyl groups with 1 to 4 carbon atoms, preferably methyl

**12.** 2-azabicyclo[2.2.1]hept-5-ene derivatives of formula VIII.

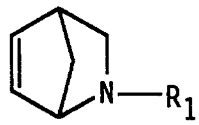

( VIII )

wherein $R_1$ is ethyl, propyl, i-propyl, allyl or butyl

**13.** 2-Azabicyclo[2.2.1]heptane derivatives of formula IX

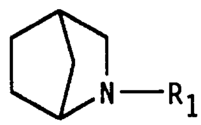

( IX )

wherein $R_1$ are the residues 2-hydroxyethyl, n-propyl, 3-methoxypropyl, n-butyl, 2-ethylhexyl, 2-hydroxyethoxyethyl, cyclohexyl, benzyl, 3-dimethylaminopropyl, 2-acetoxyethoxyethyl or 2-dimethylaminoethoxyethyl

**14.** Bis-(2-azabicyclo[2.2.1]heptyl-N-ethyl-)ether

**Claims for the following Contracting State : ES**

**1.** Process for the preparation of polyurethanes and polyepoxy resins which comprises reaction of the starting materials in the presence of 2-azabicyclo[2.2.1]hept-5-ene derivatives substituted in position 2 and, optionally, in position 3, of the general formula I,

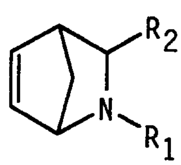

( I )

or of the corresponding 2-azabicyclo[2.2.1]heptane derivatives of the general formula II

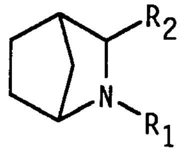

( II )

wherein:

$R_1$ are alkyl groups with 1 to 8 carbon atoms, alkenyl groups with 2 to 8 carbon atoms, cycloalkyl or alkylcycloalkyl groups with 5 to 9 carbon atoms, the benzyl group, alkylbenzyl groups with 8 to 10 carbon atoms, heteroaryl groups with one or more of the heteroatoms N, O, or S with 3 to 10 carbon atoms, $-(CH_2)_a-P$, $-(CH_2)_b-T-(CH_2)_c-U$, or $-(CH_2)_d-V-(CH_2)_e-W-(CH_2)_f-X$

wherein: a through f are 2, 3, or 4

P, U, X are equal or different and are OH, O-acyl, N-acyl, N-alkyl, N-dialkyl, the alkyl residue having 1 to 4 carbon atoms and, preferably, being methyl, or 2-azabicyclo[2.2.1]-hept-2-yl,

T, V, W are equal or different and are O, NH, or N-alkyl, the alkyl residue having 1 to 4 carbon atoms and, preferably, being methyl,

$R_2$ are hydrogen or alkyl groups with 1 to 4 carbon atoms, preferably hydrogen,

as catalysts, alone or as mixtures with other catalysts.

**2.** Process for preparing 2-azabicyclo[2.2.1]hept-5-ene derivatives substituted in position 2 and, optionally, in position 3 by reacting compounds of formula III with compounds of formula IV and by reacting the imine salt thus obtained with cyclopentadiene to yield the compounds of formula V,

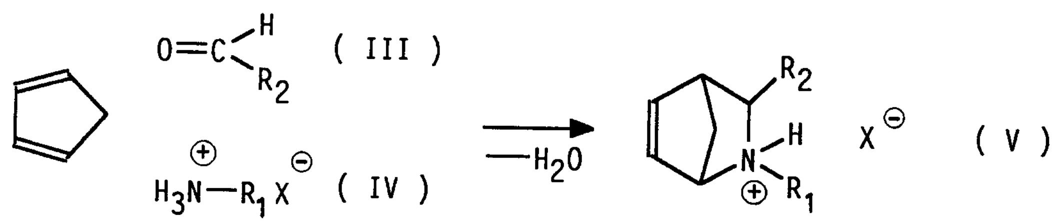

wherein:

$R_1$ represents alkyl with 3 to 8 carbon atoms in the unbranched chain and 5 to 8 carbon atoms in the branched chain, alkenyl groups with 2 to 8 carbon atoms, cycloalkyl or alkylcycloalkyl groups with 5 to 9 carbon atoms, alkyl benzyl groups with 8 to 10 carbon atoms, heteroaryl groups with one or more of the heteroatoms N, O, or S with 3 to 10 carbon atoms, $-(CH_2)_a-P$, or $-(CH_2)_b-T-(CH_2)_c-U$,

wherein: a through c are 2, 3 or 4,

P, U are equal or different and are OH, O-acyl, N-acyl, N-alkyl, N-dialkyl, the alkyl residue having 1 to 4 carbon atoms and, preferably, being methyl, or 2-azabicyclo[2.2.1]-hept-2-yl,

T is O, NH, or N-alkyl, the alkyl residue having 1 to 4 carbon atoms and, preferably, being methyl

$R_2$ represents hydrogen or alkyl groups with 1 to 4 carbon atoms, preferably hydrogen

X represents the residue of a strong acid,

preparation of the free bases by neutralization of the salts with at least equivalent amounts of a strong base or by treatment with basic exchange resins and, optionally, catalytic hydrogenation of the free bases in the presence of noble metal catalysts to yield the corresponding 2-azabicyclo[2.2.1]heptane derivatives.

**3.** Process according to claim 2, wherein salts of primary amines according to formula IV are used, which are formed in situ.

**4.** Process according to claim 2 or 3, wherein formaldehyde is used as the carbonyl compound according to formula III.

**5.** Process according to any one of the claims 2 to 4, wherein the reaction is performed in a two-phase system in a pH range of between 0 and 7, preferably between 2 and 6, and in a temperature range of between 20 and 45 °C, preferably between 10 and 45 °C, and particularly between 20 and 40 °C.

**6.** Process according to any one of the claims 2 to 5, wherein cyclopentadiene is used at a mole ratio of 0.5 : 1 to 2 : 1, relative to the iminium salt, preferably at a mole ratio of 1 : 1 to 1.5 : 1, particularly 1.1 : 1.

**7.** Process according to any one of the claims 2 to 6, wherein the hydrogenation is performed under pressure in the presence of metals of the platinum group, particularly in the presence of palladium.

**8.** Process according to any one of the claims 2 to 7, wherein, in alteration of the process defined in claim 2, the salts according to formula V which are obtained in solution during the process are directly hydrogenated.

**9.** Process according to any one of the claims 2 to 8, wherein the hydrogenation is performed at ambient or elevated temperature of between 20 and 150 °C, preferably between 50 and 100 °C, and at elevated pressure of between 1 and 15 mPa, preferably between 2 and 5 mpa.

**10.** Process for preparing azabicyclo[2.2.1]heptane derivatives by reaction of the compounds prepared according to any one of the claims 2 to 9, the substituent $R_1$ of which containing an OH- or NH-group, with acylating agents such as carboxylic acids, the anhydrides or halogenides thereof, according to equation VI

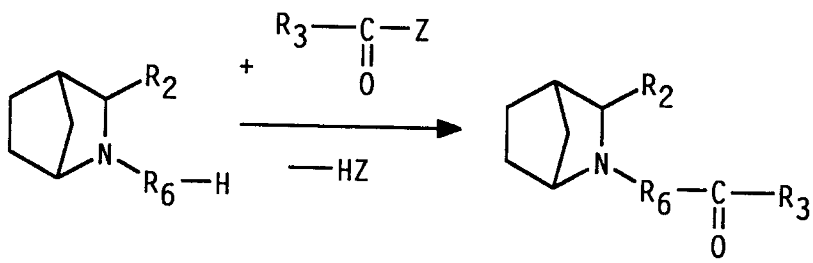

( VI )

wherein

| | |
|---|---|
| $R_6$ | is $-(CH_2)_a-V$, or $-(CH_2)_b-T-(CH_2)_c-W$, a through c being 2, 3 or 4 |
| V, W | is O or N-alkyl, the alkyl residue having 1 to 4 carbon atoms |
| T | is O, NH or N-alkyl, the alkyl residue having 1 to 4 carbon atoms and, preferably, being methyl |
| $R_2$ | is hydrogen or an alkyl group with 1 to 4 carbon atoms, |

**11.** Process for preparing azabicyclo[2.2.1]heptane derivatives by reaction of the compounds prepared according to any one of the claims 2 to 9, the substituent $R_1$ of which containing an OH group, in the presence of strong bases (particularly chlorine compounds) with halogen-containing compounds, particularly with 2-, 3-, or 4-halogen alkyl amines (particularly chlorine compounds), according to equation VII

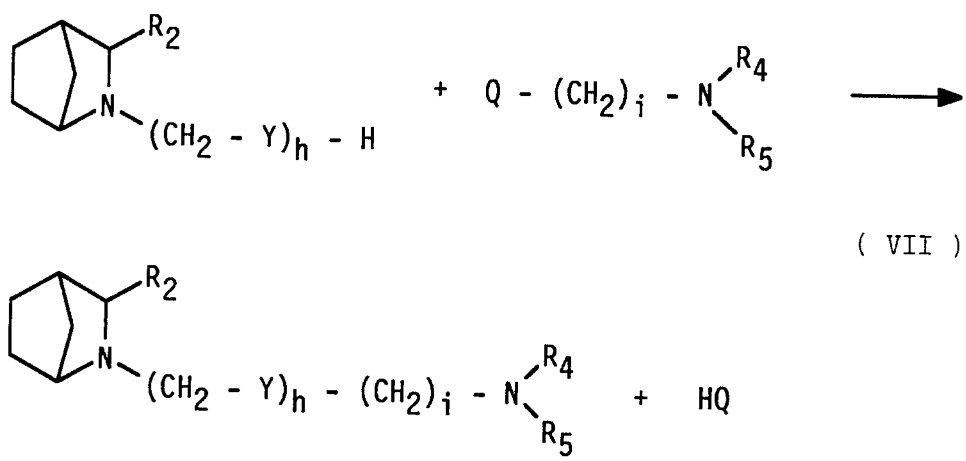

( VII )

wherein

| | |
|---|---|
| h, i | are equal or different and are 2 or 3, |
| Y | is oxygen, one or more alkoxy groups with 2 to 4 carbon atoms, NH, or N-alkyl, particularly N-methyl |
| Q | is halogen, preferably chlorine, pseudohalogen, sulfate, or sulfonate |

| | |
|---|---|
| $R_2$ | is hydrogen or alkyl groups with 1 to 4 carbon atoms, preferably hydrogen |
| $R_4$, $R_5$ | are equal or different and represent alkyl groups with 1 to 4 carbon atoms, preferably methyl |

## Revendications

### Revendications pour les Etats contractants suivants : AT, BE, CH, DE, FR, GB, IT, LI, NL, SE

**1.** Utilisation de dérivés de 2-azabicyclo[2.2.1]hept-5-ène substitués en position 2 et le cas échéant en position 3, de formule générale I

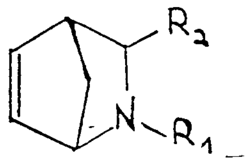

(I)

et des dérivés de 2-azabicyclo[2.2.1]heptane correspondants de formule générale II

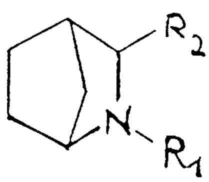

(II)

formules dans lesquelles

| | |
|---|---|
| $R_1$ | représente des groupes alkyle ayant 1 à 8 atomes de carbone, des groupes alcényle ayant 2 à 8 atomes de carbone, des groupes cycloalkyle ou alkylcycloalkyle ayant 5 à 9 atomes de carbone, le groupe benzyle, des groupes alkylbenzyle ayant 8 à 10 atomes de carbone, des groupes hétéroaryle ayant un ou plusieurs hétéro-atomes N, O ou S et 3 à 10 atomes de carbone, des groupes $-(CH_2)_a$ -P, $-(CH_2)_b$-T-$CH_2)_c$-U ou $-(CH_2)_d$-V-$(CH_2)_e$-W-$(CH_2)_f$-X<br>où a à f ont les valeurs 2, 3 ou 4, |
| P, U, X | sont identiques ou différents et représentent des groupes OH, O-acyle, N-acyle, N-alkyle, N-dialkyle - le reste alkyle ayant 1 à 4 atomes de carbone et étant de préférence un reste méthyle - ou 2-azabicyclo[2.2.1]-hept-2-yle, |
| T, V, W | sont identiques ou différents et représentent O, NH ou un groupe N-alkyle - le reste alkyle présentant 1 à 4 atomes de carbone et étant de préférence un reste méthyle - et |
| $R_2$ | représente l'hydrogène ou des groupes alkyle ayant 1 à 4 atomes de carbone, de préférence l'hydrogène, |

comme catalyseurs pour la production de polyuréthannes et de résines poly-époxyde, individuellement ou en mélange avec d'autres catalyseurs.

**2.** Production de dérivés substitués en position 2 et le cas échéant en position 3, du 2-azabicyclo[2.2.1]-hept-5-ène par réaction de composés de formule III avec des composés de formule IV et réaction du sel d'imine ainsi produit avec le cyclopentadiène pour former des composés de formule V

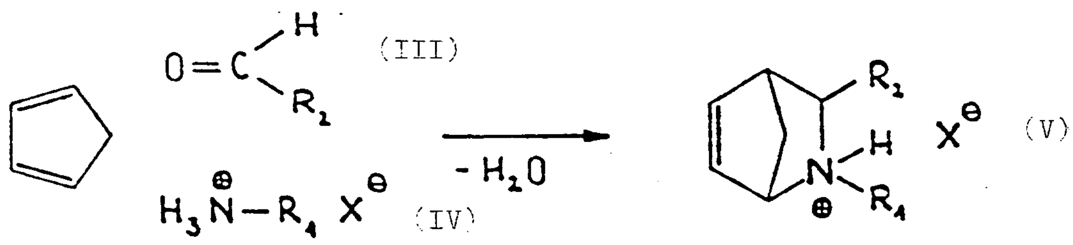


où

$R_1$ est un groupe alkyle ayant 3 à 8 atomes de carbone en chaîne non ramifiée et 5 à 8 atomes de carbone en chaîne ramifiée, des groupes alcényle ayant 2 à 8 atomes de carbone, des groupes cycloalkyle ou alkylcycloalkyle ayant 5 à 9 atomes de carbone, des groupes alkylbenzyle ayant 8 à 10 atomes de carbone, des groupes hétéroaryle ayant un ou plusieurs hétéro-atomes N, O ou S avec 3 à 10 atomes de carbone, un groupe $-(CH_2)_a-P$ ou $-(CH_2)_b-T-(CH_2)_c-U$,
où a à c ont la valeur 2, 3 ou 4,

P, U sont identiques ou différents et représentent un groupe OH, O-acyle, N-acyle, N-alkyle, N-dialkyle - le reste alkyle ayant 1 à 4 atomes de carbone et étant de préférence le reste méthyle - ou 2-azabicyclo[2.2.1]- hept-2-yle,

T représente O, un groupe NH ou N-alkyle - le reste alkyle ayant 1 à 4 atomes de carbone et étant de préférence un reste méthyle - et

$R_2$ représente l'hydrogène ou des groupes alkyle ayant 1 à 4 atomes de carbone, de préférence l'hydrogène,

X est le reste d'un acide fort,

obtention des bases libres par neutralisation des sels avec des quantités au moins équivalentes d'une base forte ou par traitement avec des résines échangeuses basiques et le cas échéant hydrogénation catalytique des bases libres en présence de catalyseurs contenant des métaux nobles pour former les dérivés correspondants du 2-azabicyclo[2.2.1]heptane.

**3.** Procédé suivant la revendication 2, caractérisé en ce qu'on utilise des sels d'amines primaires de formule IV qui sont produites in situ.

**4.** Procédé suivant la revendication 2 ou 3, caractérisé en ce qu'on utilise le formaldéhyde comme composé carbonylique de formule III.

**5.** Procédé suivant l'une des revendications 2 à 4, caractérisé en ce qu'on conduit la réaction dans un système de deux phases dans une plage de pH de 0 à 7, de préférence de 2 à 6, et dans une plage de température de 20 à 45 ° C, mieux encore de 10 à 45 ° C et notamment de 20 à 40 ° C.

**6.** Procédé suivant l'une des revendications 2 à 5, caractérisé en ce qu'on utilise du cyclopentadiène dans le rapport molaire de 0,5:1 à 2:1, par rapport au sel d'iminium, de préférence dans le rapport molaire de 1:1 à 1,5:1, notamment de 1,1:1.

**7.** Procédé suivant l'une des revendications 2 à 6, caractérise en ce qu'on conduit l'hydrogénation sous pression en présence de métaux du groupe du platine, notamment en présence de palladium.

**8.** Procédé suivant l'une des revendications 2 à 7, caractérisé en ce que dans une variante du procédé selon la revendication 2, on hydrogène directement des sels de formule V obtenus en solution au cours du procédé.

**9.** Procédé suivant l'une des revendications 2 à 8, caractérisé en ce qu'on conduit l'hydrogénation à la température ambiante ou à une température élevée dans la plage de 20 à 150 ° C, de préférence de 50 à 100 ° C et à une pression élevée dans la plage de 1 à 15 mPa (10 à 150 bars), de préférence de 2 à 5 MPa (20 à 50 bars).

**10.** Procédé de production de dérivés d'azabicyclo[2.2.1]heptane par transformation de composés produits selon l'une des revendications 2 à 9, dans lesquels le substituant $R_1$ contient un groupe OH ou un groupe NH, avec des agents acylants tels que des acides carboxyliques, leurs anhydrides ou leurs halogénures selon l'équation VI

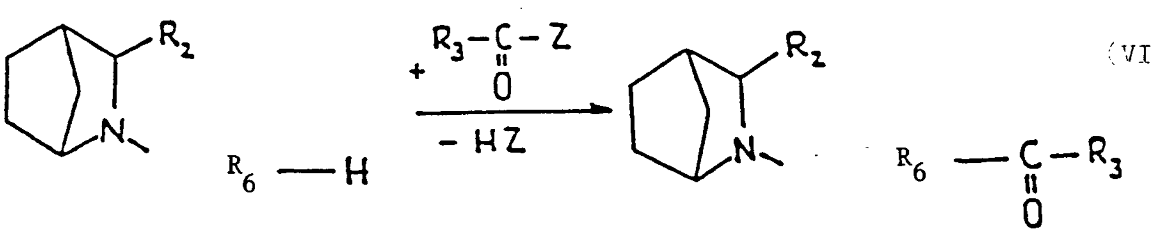


(VI)

dans laquelle

| | |
|---|---|
| $R_6$ | représente un groupe $-(CH_2)_a-V$, ou $-(CH_2)_b-T-(CH_2)_c-W$ a à c ont la valeur 2, 3 ou 4 |
| V, W | représentent O ou un groupe N-alkyle, le reste alkyle ayant 1 à 4 atomes de carbone, |
| T | représente O, un groupe NH ou N-alkyle, le reste alkyle ayant 1 à 4 atomes de carbone et étant de préférence un groupe méthyle et |
| $R_2$ | est l'hydrogène ou un groupe alkyle ayant 1 à 4 atomes de carbone. |

**11.** Procédé de production de dérivés d'azabicyclo[2.2.1]-heptane par transformation de composés préparés selon l'une des revendications 2 à 9, dans lesquels le substituant $R_1$ contient un groupe OH,

en présence de bases fortes avec des composés halogénés, notamment des 2-, 3- ou 4-halogénalkylamines (en particulier des composés chlorés) conformément à l'équation VII

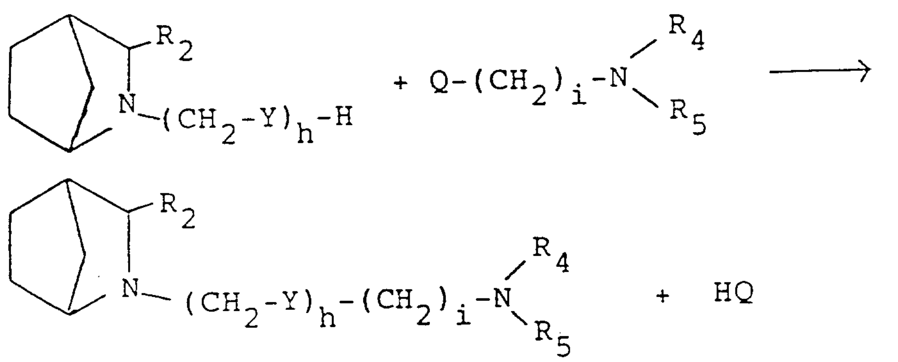

(VII)

dans laquelle

| | |
|---|---|
| h, i | sont identiques ou différents et ont la valeur 2 ou 3, |
| Y | représente l'oxygène, un ou plusieurs groupes alkoxy ayant 2 à 4 atomes de carbone, NH ou N-alkyle, notamment N-méthyle, |
| Q | est un halogène (de préférence le chlore, un pseudo-halogène, un sulfate ou un sulfonate, |
| $R_2$ | est l'hydrogène ou représente des groupes alkyle ayant 1 à 4 atomes de carbone, de préférence l'hydrogène, |
| $R_4$, $R_5$ | sont identiques ou différents et sont des groupes alkyle ayant 1 à 4 atomes de carbone, de préférence le groupe méthyle. |

**12.** Dérivés de 2-azabicyclo[2.2.1]hept-5-ène de formule VIII

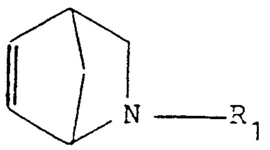

(VIII)

dans laquelle $R_1$ désigne un groupe éthyle, propyle, iso-propyle, allyle ou butyle.

**13.** Dérivés de 2-azabicyclo[2.2.1]heptane de formule

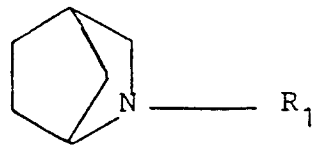

dans laquelle $R_1$ représente les restes 2-hydroxyéthyle, n-propyle, 3-méthoxypropyle, n-butyle, 2-éthylhexyle, 2-hydroxyéthoxyéthyle, cyclohexyle, benzyle, 3-diméthylaminopropyle, 2-acétoxyéthoxyéthyle ou 2-diméthylaminoéthoxyéthyle.

**14.** L'éther de bis-(2-azabicyclo[2.2.1]heptyl-N-éthyle.

**Revendications pour l'Etat contractant suivant : ES**

**1.** Procédé de production de polyuréthannes et de résines polyépoxyde, caractérisé en ce qu'on fait réagir des matières de départ en présence de dérivés de 2-azabicyclo[2.2.1]hept-5-ène, substitués en position 2 et le cas échéant en position 3, de formule générale I

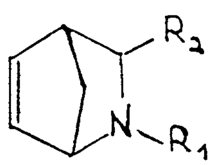

(I)

ou des dérivés correspondants de 2-azabicyclo[2.2.1]heptane de formule générale II

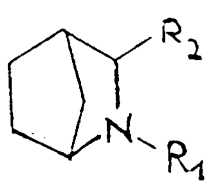

(II)

formules dans lesquelles

$R_1$ représente des groupes alkyle ayant 1 à 8 atomes de carbone, des groupes alcényle ayant 2 à 8 atomes de carbone, des groupes cycloalkyle ou alkylcycloalkyle ayant 5 à 9 atomes de carbone, le groupe benzyle, des groupes alkylbenzyle ayant 8 à 10 atomes de carbone, des groupes hétéroaryle ayant un ou plusieurs hétéro-atomes N, O ou S et 3 à 10 atomes de carbone, des groupes $-(CH_2)_a$ -P, $-(CH_2)_b$-T-$(CH_2)_c$-U ou $-(CH_2)_d$-V-$(CH_2)_e$-W-$(CH_2)_f$-X

où a à f ont les valeurs 2, 3 ou 4,

P, U, X — sont identiques ou différents et représentent des groupes OH, O-acyle, N-acyle, N-alkyle, N-dialkyle - le reste alkyle ayant 1 à 4 atomes de carbone et étant de préférence un reste méthyle - ou 2-azabicyclo[2.2.1]-hept-2-yle,

T, V, W — sont identiques ou différents et représentent O, NH ou un groupe N-alkyle - le reste alkyle présentant 1 à 4 atomes de carbone et étant de préférence un reste méthyle - et

$R_2$ — représente l'hydrogène ou des groupes alkyle ayant 1 à 4 atomes de carbone, de préférence l'hydrogène,

comme catalyseurs, individuellement ou en mélange avec d'autres catalyseurs.

**2.** Procédé de production de dérivés de 2-azabicyclo[2.2.1]hept-5-ène substitués en position 2 et le cas échéant en position 3, par réaction de composés de formule III avec des composés de formule IV et réaction du sel d'imine ainsi produit avec le cyclopentadiène pour former des composés de formule V

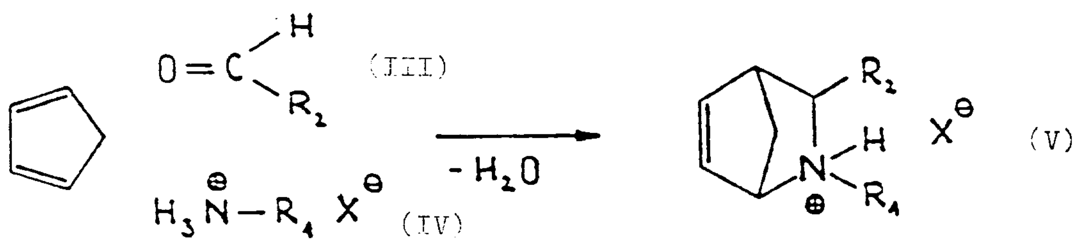

(III) (IV) (V)

formules dans lesquelles

$R_1$ — est un groupe alkyle ayant 3 à 8 atomes de carbone en chaîne non ramifiée et 5 à 8 atomes de carbone en chaîne ramifiée, des groupes alcényle ayant 2 à 8 atomes de carbone, des groupes cycloalkyle ou alkylcycloalkyle ayant 5 à 9 atomes de carbone, des groupes alkylbenzyle ayant 8 à 10 atomes de carbone, des groupes hétéroaryle ayant un ou plusieurs hétéro-atomes N, O ou S avec 3 à 10 atomes de carbone, un groupe $-(CH_2)_a-P$ ou $-(CH_2)_b-T-(CH_2)_c-U$

où a à c ont la valeur 2, 3 ou 4,

P, U — sont identiques ou différents et représentent un groupe OH, O-acyle, N-acyle, N-alkyle, N-dialkyle - le reste alkyle ayant 1 à 4 atomes de carbone et étant de préférence le reste méthyle - ou 2-azabicyclo[2.2.1]-hept-2-yle,

T — représente O, un groupe NH ou N-alkyle - le reste alkyle ayant 1 à 4 atomes de carbone et étant de préférence un reste méthyle - et

$R_2$ — représente l'hydrogène ou des groupes alkyle ayant 1 à 4 atomes de carbone, de préférence l'hydrogène,

X — est le reste d'un acide fort,

obtention des bases libres par neutralisation des sels avec des quantités au moins équivalentes d'une base forte ou par traitement avec des résines échangeuses basiques et le cas échéant hydrogénation catalytique des bases libres en présence de catalyseurs contenant des métaux nobles pour former les dérivés correspondants du 2-azabicyclo[2.2.1]heptane.

**3.** Procédé suivant la revendication 2, caractérisé en ce qu'on utilise des sels d'amines primaires de formule IV qui sont produits in situ.

**4.** Procédé suivant la revendication 2 ou 3, caractérisé en ce qu'on utilise le formaldéhyde comme composé carbonylique de formule III.

**5.** Procédé suivant l'une des revendications 2 à 4, caractérisé en ce qu'on conduit la réaction dans un système de deux phases dans une plage de pH de 0 à 7, de préférence de 2 à 6, et dans une plage de température de 20 à 45 °C, mieux encore de 10 à 45 °C et notamment de 20 à 40 °C.

**6.** Procédé suivant l'une des revendications 2 à 5, caractérisé en ce qu'on utilise du cyclopentadiène dans le rapport molaire de 0,5:1 à 2:1, par rapport au sel d'iminium, de préférence dans le rapport molaire de 1:1 à 1,5:1, notamment de 1,1:1.

**7.** Procédé suivant l'une des revendications 2 à 6, caractérisé en ce qu'on conduit l'hydrogénation sous pression en présence de métaux du groupe du platine, notamment en présence de palladium.

**8.** Procédé suivant l'une des revendications 2 à 7, caractérisé en ce que dans une variante du procédé selon la revendication 2, on hydrogène directement des sels de formule V obtenus en solution au cours du procédé.

**9.** Procédé suivant l'une des revendications 2 à 8, caractérisé en ce qu'on conduit l'hydrogénation à la température ambiante ou à une température élevée dans la plage de 20 à 150 °C, de préférence de 50 à 100 °C et à une pression élevée dans la plage de 1 à 15 MPa (10 à 150 bars), de préférence de 2 à 5 MPa (20 à 50 bars).

**10.** Procédé de production de dérivés d'azabicyclo[2.2.1]heptane par transformation de composés produits selon l'une des revendications 2 à 9, dans lesquels le substituant $R_1$ contient un groupe OH ou un groupe NH, avec des agents acylants tels que des acides carboxyliques, leurs anhydrides ou leurs halogénures selon l'équation VI

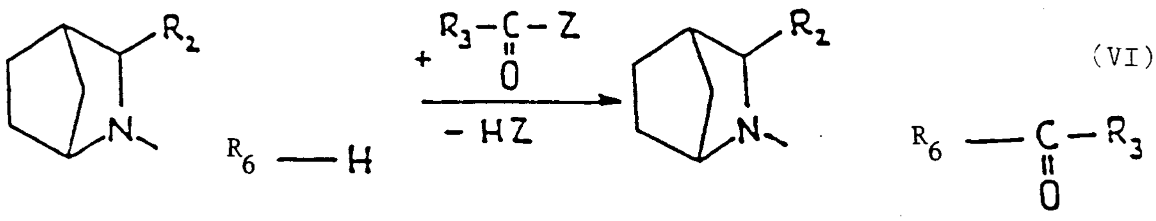

(VI)

dans laquelle

- $R_6$ représente un groupe $(CH_2)_a$-V ou $(CH_2)_b$-T-$(CH_2)_c$-W
  a à c ont la valeur 2, 3 ou 4
- V, W représentent O ou un groupe N-alkyle, le reste alkyle ayant 1 à 4 atomes de carbone,
- T représente O, un groupe NH ou N-alkyle, le reste alkyle ayant 1 à 4 atomes de carbone et étant de préférence un groupe méthyle et
- $R_2$ est l'hydrogène ou un groupe alkyle ayant 1 à 4 atomes de carbone.

**11.** Procédé de production de dérivés d'azabicyclo[2.2.1]-heptane par transformation de composés préparés selon l'une des revendications 2 à 9, dans lesquels le substituant $R_1$ contient un groupe OH,
en présence de bases fortes avec des composés halogénés, notamment des 2-, 3- ou 4-halogénalkylamines (en particulier des composés chlorés) conformément à l'équation VII

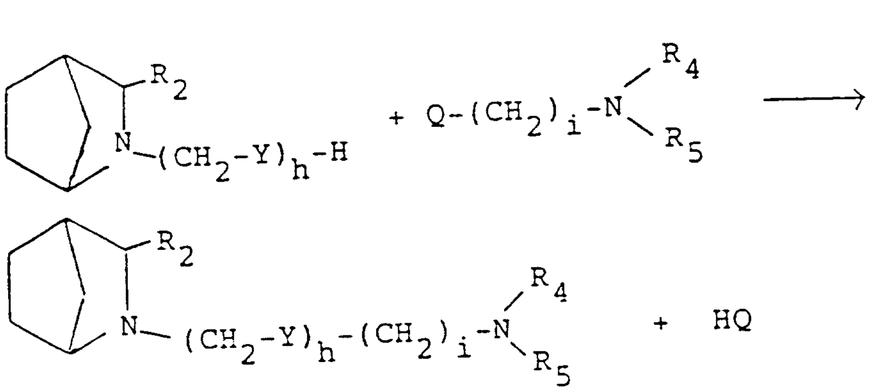

(VII)

dans laquelle

- h, i sont identiques ou différents et ont la valeur 2 ou 3,
- Y représente l'oxygène, un ou plusieurs groupes alkoxy ayant 2 à 4 atomes de carbone, NH ou N-alkyle, notamment N-méthyle,
- Q est un halogène (de préférence le chlore, un pseudo-halogène, un sulfate ou un

sulfonate,

$R_2$ est l'hydrogène ou représente des groupes alkyle ayant 1 à 4 atomes de carbone, de préférence l'hydrogène,

$R_4$, $R_5$ sont identiques ou différents et sont des groupes alkyle ayant 1 à 4 atomes de carbone, de préférence le groupe méthyle.